# EUROPEAN PATENT APPLICATION

(11) **EP 2 426 215 A1**
(43) Date of publication of application: **07.03.2012**
(21) Application number: 10175517.1
(22) Date of filing: 07.09.2010
(51) Int. Cl.: C12Q 1/68

(54) **Molecular method for the identification of mating type genes of truffles species**

(71) Applicant: INSTITUT NATIONAL DE LA RECHERCHE AGRONOMIQUE, 75338 Paris Cedex 07 (FR); Consiglio Nazionale delle Ricerche, 00185 Roma (IT)
(72) Inventor: Martin, Francis, 54280 Champenoux (FR); Murat-Furminieux, Claude, 54130 Saint Max (FR); Paolocci, Francesco, 06135 Perugia (IT); Rubini, Andrea, 06012 Citta di Castello (IT); Riccioni, Claudia, 06132 Perugia (IT); Belfiori, Béatrice, 06128, Perugia (IT); Arcioni, Sergio, 06132 Perugia (IT)
(74) Representative: Lotaut, Yacine Diaw

(57) **Abstract**

The invention concerns a method for determining the *Tuber* species and fertility of a truffle sample, said method comprising:
- identifying whether said truffle sample comprises a first nucleotide sequence associated with a first mating type idiomorph (MAT1-2) ; and
- identifying whether said truffle sample comprises a second nucleotide sequence associated with a second mating type idiomorph (MAT1-1); the fertility of said truffle sample requiring the presence of both the first and the second nucleotide sequences.

## Description

### INVENTION DOMAIN

The invention concerns the setting up of a method for the identification of truffles species and their mating type. The invention is based on the use of mating-specific nucleotide sequences.

An aim of the invention is to control the sexual reproduction of the truffles.

An additional aim of the invention is to distinguish between the truffles species.

The invention concerns the production and the control of truffle inoculated seedlings, with direct impact on the activity and interest of truffles nurseries and agro-food industry.

### STATE OF THE ART AND ENCOUNTED PROBLEM

Truffles are Ascomycete mushrooms belonging to the *Tuber* genus which live in ectomycorrhizal symbiosis with various trees. These mushrooms form fructifications generally named « truffles » which are very appreciated for their organoleptic qualities. Among these truffles species, the Périgord black truffle (*Tuber melanosporum* Vittad.), the Piémont white truffle (*Tuber magnatum* Pico) and the Burgundy Truffle (*Tuber aestivum* Vittad.) are the most appreciated species by the gastronomes and have a large cultural and economic impact. Indeed, their price can vary between 200-400 euro/kg for the Burgundy truffle, 400-1,000 euro/kg for the Périgord truffle and 2,000-6,000 euro/kg for the Piémont white truffle. This very high price is, among other things, the consequence of a high decrease of their production over the last decades. In view to preserve these species and to increase their production, a protocol permitting to inoculate host plantlets, with each of the previously mentioned truffle species has been made to be exploited by truffles nurseries.

This protocol consists to inoculate first young oak, hazel, pin or other host plantlets by mean of spores from several truffles. Each truffle used as spore donor is morphologically controlled to avoid contamination with undesired species. Alternatively, host plants can be inoculated with in vitro grown mycelia.

Then, the development of mycorrhizas on the inoculated plants is controlled 1) to exclude plants that either do not develop mycorrhizas or harbour mycorrhizas from contaminating fungal species according to morphological criteria and 2) to assess whether the percentage of mycorrhization is sufficient. If the level of mycorrhization is sufficient and few contamination are detected the mycorrhized plantlets can be sold. Mycorrhized plantlets means that plantlet roots have been colonized by truffle mycelium and that truffle mycelium and plantlet live in a symbiotic relationship the one with the other.

When these verifications have been met, these mycorrhized plantlets are planted in an orchard. After 5 to 8 years of culturing, it might be obtained truffles of the *Tuber* species that has been inoculated.

The discrimination of *Tuber* vs non *Tuber* mycorrhizas by morphological criteria is often an easy task. However, the discrimination among mycorrhizas of the different *Tuber* species is sometime impossible, such as the typing of mycorrhizas formed by the Périgord Black truffles from those formed by the Chinese black truffles. Mistakes are possible mainly with the Chinese black truffle *Tuber indicum.* Indeed this species is morphologically very similar to the Périgord Black truffle and sometimes their morphological discrimination is impossible. *T. indicum* is a truffle that presents organoleptic qualities lesser pronounced than the ones presented by *T. melanosporum.*

Moreover, despite advances in the inoculation methodology and cultural improvements, some trees and plantations are still sterile without producing truffles. So the quantity of truffles that can be produced by a truffle plant is unpredictable.

### SUMMARY OF THE INVENTION

The inventors have discovered that truffles, and in particular *T. melanosporum* and *T. magnatum* are obligate outcrossing fungi, also known as heterothallic fungi. These mushrooms are self-incompatible and therefore need the presence of a partner of opposite mating type to reproduce themselves.

The inventors have discovered that to ensure sexual reproduction of truffle species, the mating between two sexually compatible mycelia is needed. These two mycelia differ for the mating type genes in their genome.

Mating type genes or *MAT* genes are the master loci controlling sexual reproduction and development in fungi.

Sexual reproduction in Ascomycetes filamentous fungi is controlled by two different *MAT* genes that established sexual compatibility: one *MAT* gene codes for a protein with an alpha box domain (MAT1-1-1), whereas the other encodes a high mobility group (HMG) protein (MAT1-2-1). In heterothallic Ascomycetes, there are strains that have only one of these genes in their genome, the other mating type gene is present in different strains at the same site on the chromosome, namely MAT locus. Because this locus contains different genes and other highly divergent sequence, the two versions of a *MAT* locus (MAT1-1 and MAT1-2) are not called allelic and are called idiomorphs (Metzemberg & Glass, 1990, Bioessays 12: 53-59).

To gain insight into the truffle reproduction system, the inventors have tried to identify the nucleotide sequences of the *MAT* locus in each of the most famous truffles species taking advantage from the fact that the genome of the Périgord black truffle has been recently deciphered. Thus, the inventors have first tried to identify, in *T. melanosporum,* the nucleotide sequences corresponding to the two mating type genes. Indeed, due to the high nucleotide sequence divergences between mating type genes within Ascomycetes, the inventors have not immediately found the nucleotide sequences corresponding to the two mating type genes.

The inventors have first screened the sequenced *T. melanosporum* genome for the presence of candidate genes encoding for the protein containing the HMG and the alpha-box domain using as queries mating type genes from other Ascomycetes. Following this approach, the inventors have discovered in the *T. melanosporum* genome three nucleotides sequences (three genes: GSTUMT00001090001, GSTUMT00000587001 and GSTUMT00008644001) that have homologies with the HMG containing mating type gene of other Ascomycetes. On the contrary, no *T. melanosporum* genes corresponding to the alpha-box mating type genes of other Ascomycetes has been detected, suggesting that *T. melanosporum* is indeed a heterothallic species. For each of the three HMG genes identified in *T.melanosporum* genome, the inventors have designed a specific primer pair to verify their presence in the other *T. melanosporum* strains. Embracing the hypothesis of heterothallism, the putative HMG- mating type gene cannot be present in all *T. melanosporum* strain, since some strains should be of the opposite mating type. Indeed, this prediction was confirmed, as one of the three HMG containing genes (GSTUMT00001090001) was not found in all strains tested. Thus, this gene has been identified as the first mating type gene of *T. melanosporum,* namely *TmelMAT1-2-1.* The HMG box of this first mating type of *T. melanosporum* has as nucleotide sequence the nucleotide sequence SEQ ID N°1.

Having mapped the position of *TmelMAT1-2-1* on the *T. melanosporum* sequenced genome, the inventors have then designed new primers on its flanking regions to amplify the entire idiomorphic sequence both from strains with and without the *TmelMAT1-2-1* gene. In the light of heterothallism in fact, the 5' and 3' regions flanking the two putative idiomorphic structures are expected to be conserved between strains of opposite mating type. Following this approach, the second *MAT* locus (MAT1-1) has been cloned and then sequenced from a strain that lacked the *TmelMAT1-2-1* gene.

Sequence analysis confirmed the identification of the *MAT* idiomorph (MAT1-1) containing a second mating type gene, namely *TmelMAT1-1-1,* said second mating type gene encodes for a putative alpha-box containing transcription factor showing appreciable sequence similarity with the alpha box mating type genes from other Ascomycetes. This alpha box specific to the second mating type gene of *T.melanosporum* has as nucleotide sequence the nucleotide sequence SEQ ID N°2.

Overall, following the above mentioned approaches both MAT idiomorphic regions of *T.melanosporum* have been identified. The first mating type idiomorph MAT1-2 (*TmeIMAT1-2)* corresponds to the nucleotide sequence SEQ ID N°3. The second mating type idiomorph MAT1-1 (*TmeIMAT1-1*) corresponds to the nucleotide sequence SEQ ID N°4.

The inventors have determined a first genomic region comprising the first mating type gene MAT1-2-1 (or "the first mating type idiomorph MAT1-2") and a second genomic region comprising the second mating type gene MAT1-1-1 (or "the second mating type idiomorph MAT1-1 "). Each of these genomic regions comprises also flanking regions that are highly conserved.

The first genomic region has as nucleotide sequence the nucleotide sequence SEQ ID N°5 and the second genomic region has as nucleotide sequence the nucleotide sequence SEQ ID N°6.

By the way of the above identified nucleotide sequences SEQ ID N°1, SEQ ID N°2, SEQ ID N°3, SEQ ID N°4, SEQ ID N°5 and SEQ ID N°6 , the inventors were able to implement an identification method of *T.melanosporum.* But others nucleotide sequences specifics to the two mating type gene of *T.melanosporum* can be used.

The invention permits to differentiate truffles species between them. In particularly, the invention permits to differentiate Chinese truffle (*Tuber indicum* Cook & Massee) with regard to the Périgord Black truffle (*T. melanosporum*).

The invention permits therefore to avoid contamination of plantlets with *T. indicum.* The invention permits to ban the presence of *T. indicum* in the plantlets that will be planted in Europe.

The invention permits also to differentiate other truffles species between them, such as *T. melanosporum, T. magnatum, T. aestivum.*

By the way of the above identified nucleotide sequences, the inventors were also able to screen mycorrhizal plants and determine whether they sustain *T. melanosporum* mycorrhizas of one or both mating types on their roots. More precisely, the method is based on the amplification with mating-type *T. melanosporum* specific primers of DNA isolated from randomly selected pools of *T. melanosporum* mycorrhizas. If the plants harbouring both mating types, the proportion of each mating type can be measured by performing a quantitative PCR analysis or by analyzing randomly selected single ectomycorrhizas and counting the proportion of mycorrhizas harbouring the first or the second mating type.

Likewise, by the way of the above identified nucleotide sequences, the inventors were able to screen *in vitro* cultivated *T.melanosporum* mycelia.

It is also possible to guaranty to the truffle cultivators:
- the mating type(s) of strains present on mycorrhizal plantlets. The methods, in fact, permits to check whether a host plant harbours strains of only one or both mating types of a given *Tuber* species.
- host plant selection according to the mating type of the fungus;
- the setting up of truffle plantations in which the presence of strains of both mating types of a given *Tuber* species is spatially and numerically balanced in the ground. This can be achieved by using plants harbouring strains of both mating type in their roots or by outplanting close each other host plants that harbour in their roots strains of opposite mating type to promote, in open field conditions, the contact and mating between sexually compatible mycelia ;

- to control the presence and the distribution of strains of different mating type in previously realized truffle plantations as well as in natural truffle sites to induce *ex novo* or rescue the truffle production. These analyses permit, in fact, to test whether in a given truffle field the presence of strains of both mating type is spatially and numerically balanced. If not, in order to favour mating between sexually compatible strains, mycorrhized plants harbouring strains with the mating type resulted to be absent or less abundant can be selected and outplanted;
- to control the mating type of mycelia and/or other inocula used for host plant inoculation.

Each mating type gene of a given truffle species can be found with the same method as the one disclosed for obtaining the nucleotide sequence of the *TmeIMATI-2-1* and *TmelMAT1-1-1* as above indicated.

For a given *Tuber* species, it is wanted strains that comprise a first nucleotide sequence that hybridizes with the HMG containing mating type gene of other Ascomycetes. Then, from the flanking regions of this first nucleotide sequence, it is wanted primers in view to amplify a nucleotide sequence corresponding to the second mating type gene of this same given *Tuber* species for the other strains that not contain the first mating type gene.

In an embodiment, each mating type gene of a given truffle species can be identified with the help of parts of nucleotide sequences taken from the first genomic region of *T.melanosporum* and from the second genomic region of *T.melanosporum.* Each mating type gene of a given truffle species can also be identified with the help of parts of nucleotide sequences taken from the first mating type gene of *T.melanosporum* and of the second mating type gene of *T.melanosporum.* These nucleotide sequences can be used as pairs of primers to amplify respectively a first mating type gene and a second mating type gene of a given truffle species.

It is also possible to use the nucleotide sequences of the HMG specific box of *T.melanosporum* and of the alpha specific box of *T.melanosporum* as probes to identify the nucleotide sequences of a first mating type gene and a second mating type gene of a given truffle species.

The invention is used to certify the truffle species, to control fructifications of these species, the identity (and mating type) of their *in vitro* cultured and/or soil living mycelia and mycorrhizas.

The invention permits also to verify the mating type in plantlets inoculated with *T. melanosporum, T. magnatum* and *T. aestivum* which will be sold in the future, and to ensure the presence of mycelia of the two mating types in view to optimize truffles production.

The invention permits to certify the presence of compatible mycelia at the root system level.

The invention also permits to increase fruiting and hence truffle production by controlling *a priori* the presence of mating types on mycorrhized plants used to set a truffle orchard. Also, as reported above, the invention would allow the rescue of man-made and natural plantations by enriching these sites for the presence of strains harbouring the less abundant (or absent) mating type through the introduction of mycorrhized plants specifically selected for the mating type of their fungal partner.

Thus, it is possible to certify to a truffle cultivator the mating type(s) of the truffle strains on the roots of the host plants.

The invention has as object a method for determining the *Tuber* species of a truffle sample, said method comprising identifying whether said truffle sample comprises at least one nucleotide sequence chosen among a first nucleotide sequence associated with a first mating type idiomorph (MAT1-2) and a second nucleotide sequence associated with a second mating type idiomorph (MAT1-1); both nucleotide sequence being specific to a given *Tuber* species.

By truffle sample it is intended that it comprises truffle fructification sample and/or truffle mycelium sample and/or mychorrized plant sample and/or a truffle producing soil sample.

The first nucleotide sequence can be at least one part of the nucleotide sequence SEQ ID N°5. In an example, the first nucleotide sequence can be the nucleotide sequence SEQ ID N°5, or a part of the nucleotide sequence SEQ ID N°5. For example, it can be the nucleotide sequence SEQ ID N°3 or the nucleotide sequence SEQ ID N°1.

The second nucleotide sequence can be at least one part of the nucleotide sequence SEQ ID N°6. In an example, the second nucleotide sequence can be the nucleotide sequence SEQ ID N°6 or a part of the nucleotide sequence SEQ ID N°6. For example, it can be the nucleotide sequence SEQ ID N°4 or the nucleotide sequence SEQ ID N°2.

The nucleotide sequence SEQ ID N°5 is a first genomic region of *T. melanosporum* that comprises the first *T. melanosporum* mating type gene *Tme*/MAT1-2-1 or SEQ ID N°3 and the nucleotide sequence SEQ ID N°6 is a second genomic region of *T.melanosporum* that comprises the second *T. melanosporum* mating type gene *Tme*/MAT1-1-1 or SEQ ID N°4.

The invention provides also that the method further comprises determining the fertility of said truffle sample, using the identification in said truffle sample of both the first nucleotide sequence associated with the first mating type idiomorph (MAT1-2) and the second nucleotide sequence associated with a second mating type idiomorph (MAT1-1), the fertility of said truffle sample requiring the presence of both the first and the second nucleotide sequences.

The first nucleotide sequence may comprises at least one part of the nucleotide sequence SEQ ID N°5. In an embodiment, the first sequence may also comprises the nucleotide sequence SEQ ID N°5, or the nucleotide sequence SEQ ID N°3 or the nucleotide sequence SEQ ID N°1. The second nucleotide sequence may comprises at least one part of the second nucleotide sequence SEQ ID N°6. In an embodiment, the second nucleotide sequence may comprises the nucleotide sequence SEQ IDN °6 or the nucleotide sequence SEQ ID N°4 or the nucleotide sequence SEQ ID N°2.

The method may comprises the use of a first pair of oligonucleotide primers to determine the presence of the first mating type idiomorph (MAT1-2) in said truffle sample, each primer of the first pair of oligonucleotide primers comprising from 5 to 50, and preferentially from 10 to 30 successive nucleotides of the first nucleotide sequence. The first nucleotide sequence can be the nucleotide sequence SEQ ID N°5. In an embodiment, the first nucleotide sequence may comprises the nucleotide sequence SEQ ID N°3 or the nucleotide sequence SEQ ID N°1.

A forward primer of this first pair of oligonucleotide primers may comprises the nucleotide sequence SEQ ID N°7. The nucleotide sequence SEQ ID N°7 is taken from the nucleotide sequence SEQ ID N°1. Other specific nucleotide sequences can be used, as the nucleotide sequence SEQ ID N°11 or the nucleotide sequence SEQ ID N°13 that are parts of the nucleotide sequence SEQ ID N°5.

A reverse primer of this first pair of oligonucleotide primers may comprises the nucleotide sequence SEQ ID N°8. The nucleotide sequence SEQ ID N°8 is taken from the flanking region of the nucleotide sequence SEQ ID N°1. Other specific nucleotide sequences can be used, as the nucleotide sequence SEQ ID N°12 or the nucleotide sequence SEQ ID N°14 that are parts of the nucleotide sequence SEQ ID N°5.

The nucleotide sequence SEQ ID N°7 and the nucleotide sequence SEQ ID N°8 serve to amplify a part of the nucleotide sequence SEQ ID N°1.

The nucleotide sequence SEQ ID N°11 and the nucleotide sequence SEQ ID N°12 serve to amplify the nucleotide sequence SEQ ID N°3.

The nucleotide sequence SEQ ID N°13 and the nucleotide sequence SEQ ID N°14 serve to amplify the nucleotide sequence SEQ ID N°5.

The method may comprises the use of a second pair of oligonucleotide primers to determine the presence of the second mating type idiomorph (MAT1-1) in said truffle sample, each primer of the second pair of oligonucleotide primers comprising from 5 to 50, and preferentially from 10 to 30 successive nucleotides of the second nucleotide sequence. As indicated above, the second nucleotide sequence can be the following nucleotide sequences SEQ ID N°2, SEQ ID N°4 or SEQ ID N°6.

A forward primer of this second pair of oligonucleotide primers may comprises the nucleotide sequence SEQ ID N°9. The nucleotide sequence SEQ ID N°9 is taken from the nucleotide sequence SEQ ID N°2. Other specific nucleotide sequences can be used, as the nucleotide sequence SEQ ID N°11 or the nucleotide sequence SEQ ID N°13.

A reverse primer of the second pair of oligonucleotide primers may comprises the nucleotide sequence SEQ ID N°10. The nucleotide sequence SEQ ID N°10 is taken from the nucleotide sequence SEQ ID N°2. Other specific nucleotide sequences can be used, as the nucleotide sequence SEQ ID N°12 or the nucleotide sequence SEQ ID N°14.

The nucleotide sequence SEQ ID N°9 and the nucleotide sequence SEQ ID N°10 serve to amplify a part of the nucleotide sequence SEQ ID N°2.

The nucleotide sequence SEQ ID N°11 and the nucleotide sequence SEQ ID N°12 serve to amplify the nucleotide sequence SEQ ID N°4.

The nucleotide sequence SEQ ID N°13 and the nucleotide sequence SEQ ID N°14 serve to amplify the nucleotide sequence SEQ ID N°6.

The detection of the presence of at least one part of the first nucleotide sequence associated with a first mating type idiomorph of a *Tuber* species and of at least one part of a second nucleotide sequence associated with the second mating type idiomorph of the same *Tuber* species could be done with the help of usual molecular biology tools wheel known from one of ordinary skill in the art.

For example, the detection of this presence can be done in labelling a genomic marker or a specific primer and in hybridizing the genomic marker or the specific primer to an amplified DNA sequence. The marker or the primer can be at least one part of one of the two nucleotide sequences associated with a mating type idiomorph of *T.melanosporum* or an other given *Tuber* species.

The detection of this presence could be done also in labelling cDNA which are able to be detected by at least one part of a first nucleotide sequence associated with a first mating type idiomorph of a *Tuber* species and by at least one part of a second nucleotide sequence associated with a second mating type idiomorph of the same *Tuber* species.

The invention has also as object a nucleotide sequence associated with a first mating type idiomorph (MAT1-2) of a *Tuber* species comprising the nucleotide sequence SEQ ID N°5. The nucleotide sequence SEQ ID N°5 is the first genomic region of *T. melanosporum* that comprises the first mating type gene of *T.melanosporum* and the HMG specific box of *T. melanosporum.*

The invention has also as object a nucleotide sequence associated with a second mating type idiomorph (MAT1-1) of a *Tuber* species comprising the nucleotide sequence SEQ ID N°6. The nucleotide sequence SEQ ID N°6 is the second genomic region of *T. melanosporum* that comprises the second mating type gene of *T.melanosporum* and the alpha specific box of *T. melanosporum.*

The invention has also as object a first pair of oligonucleotide primers associated with a first mating type idiomorph (MAT1-2-1) of a *Tuber* species, wherein each primer comprises from 5 to 50, for preference from 10 to 30 successive nucleotides of the nucleotide sequence SEQ ID N°5.

In an embodiment, this first pair of oligonucleotides primers comprises a forward primer with as nucleotide sequence the nucleotide sequence SEQ ID N°7 and a reverse primer with as nucleotide sequence the nucleotide sequence SEQ ID N°8.

Other forward primers can be used with as nucleotide sequences the nucleotide sequence SEQ ID N° 11 or the nucleotide sequence SEQ ID N°13.

Other reverse primers can be used with as nucleotide sequences the nucleotide sequence SEQ ID N°12 or the nucleotide sequence SEQ ID N°14.

The invention has also as object a second pair of oligonucleotide primers associated with a second mating type idiomorph (MAT1-1-1) of a *Tuber* species, wherein each primer comprises from 5 to 50, for preference from 10 to 30 successive nucleotides of the nucleotide sequence SEQ ID N°6.

In an embodiment, this second pair of oligonucleotides primers comprises a forward primer with as nucleotide sequence the nucleotide sequence SEQ ID N°9, and a reverse primer with as nucleotide sequence the nucleotide sequence SEQ ID N°10.

Other forward primers can be used with as nucleotide sequences the nucleotide sequence SEQ ID N°11 or the nucleotide sequence SEQ ID N°13.

Other reverse primers can be used with as nucleotide sequences the nucleotide sequence SEQ ID N°12 or the nucleotide sequence SEQ ID N°14.

The invention can provides also the development of fast and easy to use a kit to determinate the fertility of a truffle sample, like truffle fructification sample, truffle mycelium sample, mycorrhized plant sample or truffle-producing soil sample; a kit being able to be used even by unspecialized persons. Indeed, it is possible to create a "two in one" kit allowing the identification of the two mating type in a single reaction. The idea is to merge the findings realized by the inventors with the expertise of nanotechnologists to realize such a kit.

The invention has also as object a kit for determining the fertility of a truffle sample, like truffle fructification sample, truffle mycelium sample, mycorrhized plant sample or truffle-producing soil sample, the kit comprising:
- the pair of oligonucleotide primers associated with a first mating type idiomorph (MAT1-2-1) of a *Tuber* species, as indicated above, and
- the pair of oligonucleotide primers associated with a second mating type idiomorph (MAT1-1-1) of a *Tuber* species, as indicated above.

Means for amplifying a nucleotide sequence are also included in this kit. This kit can be use also to identify the truffle species between them.

The nucleotides sequences SEQ ID N°1, SEQ ID N°2, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13 and SEQ ID N°14 are nucleotides sequences that are specifics to the species *T. melanosporum.* But others nucleotides sequences specifics to others *Tuber* specie could be used.

### BRIEF DESCRIPTION OF THE FIGURES

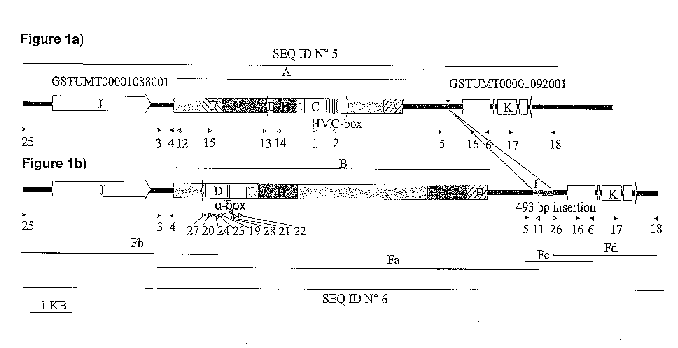

Figures 1 a and 1 b represent schematics representations of *Tuber melanosporum* mating type locus MAT1-2-1 and *Tuber melanosporum* mating type locus MAT1-1-1, respectively.
   A (SEQ ID N°3) and B (SEQ ID N°4) represent respectively the nucleotide sequence for the first mating type idiomorph of *T.melanosporum, Tme*/*MAT* 1-2, and for the second mating type idiomorph of *T.melanosporum, Tme*/*MAT* 1-1. The black lines indicate the common flanking regions. C (SEQ ID N°1) and D (SEQ ID N°2) arrows represent respectively the nucleotide sequence for the HMG box (mating type gene *Tme*/MAT 1-2-1) and for the alpha box (mating type gene *Tme*/MAT 1-1-1). An additional putative ORF was detected in the MAT1-2 idiomorph (E arrowed box; gene model: GSTUMT00001089001). The F, G and H boxes represent regions sharing sequence similarities between idiomorphs. The 45° grid pattern indicates the opposite orientation of similar sequences within and between idiomorphs. The flanking region downstream the MAT1-1 idiomorph contains a 493 bp long insertion used to design a backward primer to specifically amplify the MAT1-1 idiomorph (I Box). The arrowed boxes J and K indicate the gene models: GSTUMT00001088001 and GSTUMT00001092001, conserved in the regions flanking the idiomorphs.
   The small black, white and grey arrows indicate the position of common primers or MAT1-1 and MAT1-2 idiomorph-specific primers, respectively. Primers numbers are as in table 1. The black lines at the bottom of the figure 1 indicate the PCR amplicons (Fa, Fb, Fc and Fd) assembled to give the entire MAT1-1-1 contig (15.144 bp).

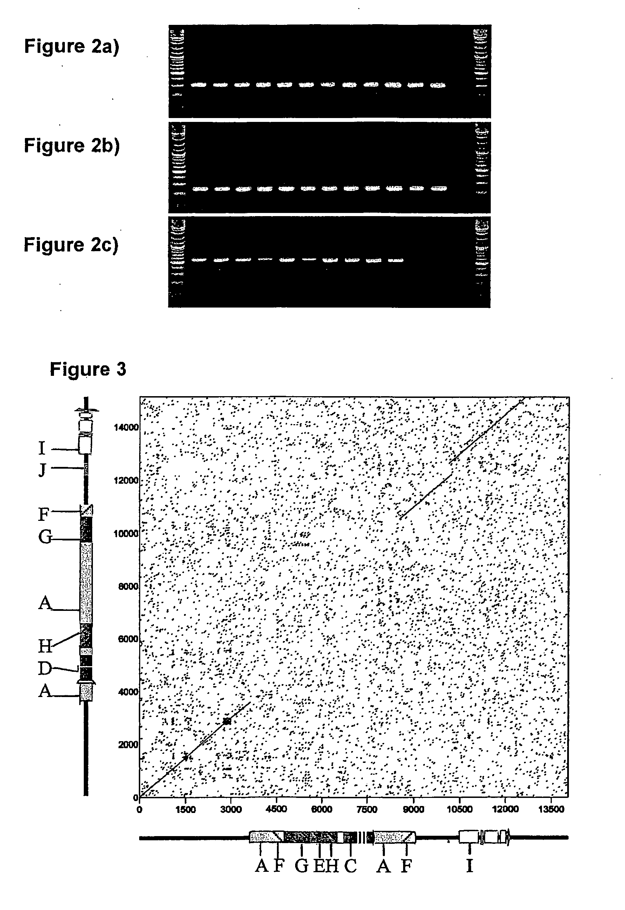

Figures 2a, 2b and 2c represent PCR amplification of three HMG-domain encoding genes in *T. melanosporum.*
   PCR amplifications were performed with the primer pair's p7/p8, p9/p10 and p1/p2 designed on the gene models GSTUMT00008644001 (a), GSTUMT00000587001 (b) and GSTUMT00001 090001 (c), respectively.
   Each PCR was performed with genomic DNA from the following ascocarps: lanes 2-13: me443, me448, me458, me459, me66, me67, me68, me300, me303, me225, me206, me151; lane 14: negative control (no DNA template); lanes 1 and 15: DNA ladder mix (Fermentas).
Figure 3 represents a dot-plot comparison of MAT1-1 and MAT1-2 DNA sequences highlighting the extent of the idiomorphic region in each mating type and the repeated sequences. The idiomorphic regions are represented as in figures 1 a and 1 b.
Figures 4a and 4b represent amino acid alignment of conserved regions of *T. melanosporum* MAT proteins with those from others Ascomycetes.

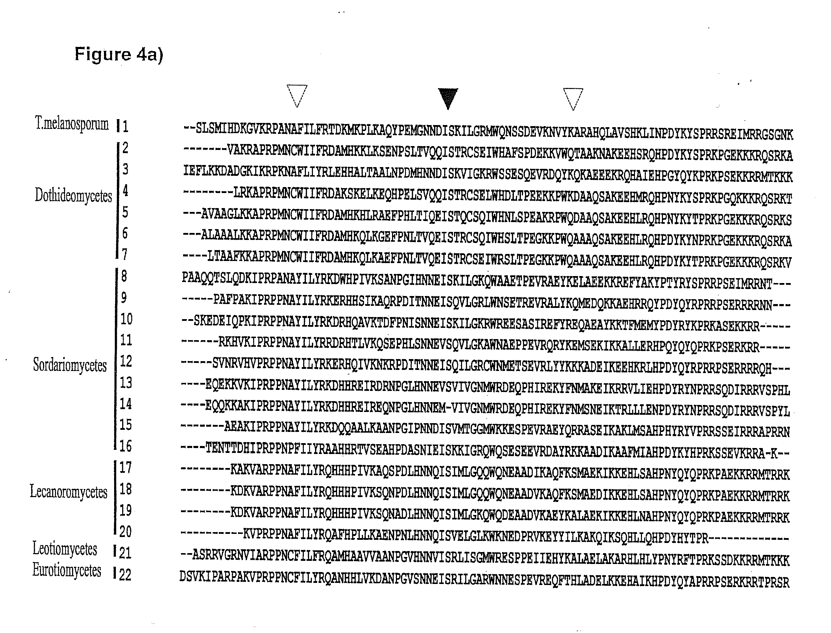

Figure 4a) represents an amino acid alignment of conserved HMG domain of the following species: 1) *Tuber melanosporum;* 2) *Leptosphaeria maculans* (AA037761); 3) *Mycosphaerella graminicola* (AAL30836); 4) *Pyrenophora teres* (AAY35017); 5) *Cochliobolus sativus* (AAF87724); 6) *Dendryphiella sp.* (AAR83920); 7) *Alternaria alternata* (BAA75908); 8) *Magnaporthe grisea* (BAC65094); 9) *Gibberella fujikuroi* (AAC71056); 10) *Ceratocystis eucalypti* (AAF00498); 11) *Isaria tenuipes* (BAC67543); 12) *Colletotrichum musae* (CAD59615); 13) *Sordaria brevicollis* (CAB63222); 14) *Neurospora crassa* (AAA33598); 15) *Podospora anserina* (CAA45520); 16) *Cryphonectria parasitica* (AAK83343); 17) *Xanthoria flammea* (CAl59780); 18) *Xanthoria elegans* (CAl59778); 19) *Xanthoria polycarpa (*CAl59768); 20) *Cladonia galindezii* (AAT48651); 21) *Pyrenopeziza brassicae* (CAA06843); 22) *Penicillium chrysogenum* (CAP 17333).

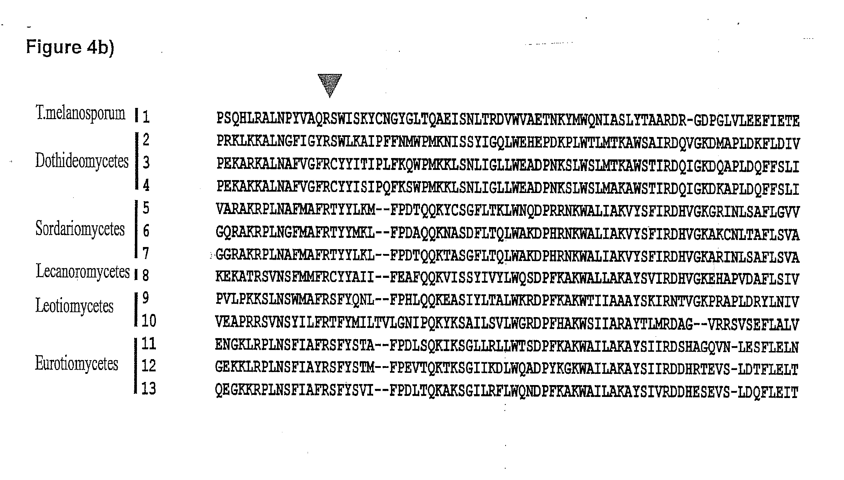

Figure 4b) represents an amino acid alignment of conserved α-box region of the following species: 1) *Tuber melanosporum;* 2) *Phaeosphaeria nodorum* (XP_001791062); 3) *Stemphylium callistephi* (AAR04468); 4) *Alternaria brassicicola* (AAK85542); 5) *Gibberella avenacea* (CAD59608); 6) *Fusarium poae* (CAD59610); 7) *Colletotrichum musae* (CAD59611); 8) *Xanthoria polycarpa* (CA159771); 9) *Pyrenopeziza brassicae* (CAA06844); *10) Botryotinia fuckeliana* (XP_001546438); 11) *Ajellomyces capsulatus* (AB026868); 12) *Penicillium marneffei* (ABC68484); 13) *Neosartorya fischeri* (XP_001263836).
   Ascomycete families are indicated with vertical bars. The position of *T*. *melanosporum* specific introns and those conserved in most of the Ascomycetes are indicated by black and white triangles respectively. In the α-box region alignment, the grey triangles indicate the position of an intron conserved in some Ascomycetes but absent in *T. melanosporum*

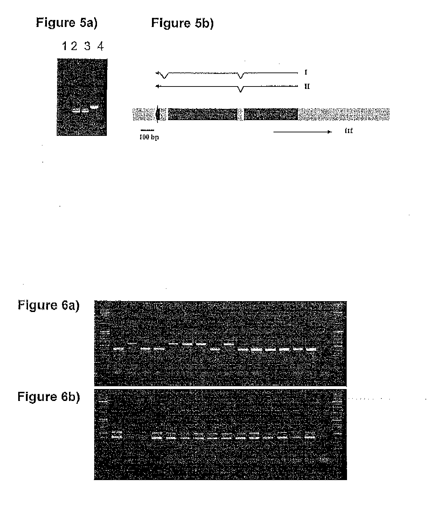

Figures 5a and 5b represent MAT1-1 gene structure as inferred from RT-PCR and RACE analyses. Figure 5a) represents PCR amplification with primers p27 and p28; Lane 1 is a 1 Kb DNA ladder (Fermentas). Lanes 2 and 3 are respectively cDNA from MAT1-1 ascocarps me206 and me151. Lane 4 is a genomic DNA from me206. Figure 5b) represents a schematic representation of MAT1-1 gene structure (dark boxes) and the two different cDNA detected (I and II). The putative antisense transcript (III) is also reported.
Figures 6a and 6b represent respectively a multiplex PCR amplification of DNA from ascocarps and a purified asci with mating type specific primers SEQ ID N°7 (p1); SEQ ID N°8 (p2); SEQ ID N°9 (p19); SEQ ID N°10 (p20).
   Lanes 1 and 18: DNA ladder mix (Fermentas); lanes 2-16: ascocarps me442, me443, me444, me445, me448, me458, me459, me69, me66, me71, me222, me182, me226, me334, me335; lane 17: negative control (no DNA template).

### EXPERIMENTATIONS

### 1. Materials and Methods

### 1.1. PCR amplification and sequencing

The primers' sequences and annealing sites on or near the *MAT* locus are reported in Table 1 and Fig. 1. PCR amplifications were performed in a 50-µl mixture containing 16 mM (NH₄)₂SO₄, 67 mM Tris-HCl (pH 8.8), 0.01 % Tween-20, 2.5 mM MgCl₂, 0.2 mM of each dNTP, 10 nM of each primer, 1 U of EuroTaq polymerase (Euroclone, Milano, Italy) and 20 ng of DNA. DNA comes from ascocarps and pools of spores that were isolated according to Paolocci et. al (2006) (complete the references). PCR reactions were performed in a 9700 PCR system (Applied Biosystems, Foster City, CA, USA) with the following thermal profile: 2 min at 94°C followed by 35 cycles of denaturation at 94°C for 30 s, annealing at 55-60°C for 30 s, extension at 72°C for 30 s to 3 min according to the length of the expected amplicon, and a final extension at 72°C for 7 min. Long-range PCR amplification of the *MAT1-1* region (about 10 Kb) was performed using LA Taq DNA polymerase (TaKaRa Bio Inc., Otsu, Japan) and a 2-step thermal profile: 30 s at 94°C, 30-40 cycles of denaturation at 94°C for 30 s, annealing/extension at 68°C for 15 min, and a final extension for 7 min at 72°C. The analysis of simple sequence repeat (SSR) loci was performed according to Riccioni et al. (2008) New Phytologist 180: 466-478.

The PCR amplicons were purified using the EuroGold Cycle-Pure PCR purification kit (Euroclone, Milano, Italy) or cloned into the pGEM T-easy vector (Promega, Madison Wl, USA) according to the manufacturer's instructions. Sequences were obtained using the BigDye sequencing kit (Applied Biosystems) according to the manufacturer's protocol. Sequence assembly was performed using Bioedit software v. 7.0.9 (Hall, 1999 Nucleic Acids Symposium. 41: 95-98). Primers were designed with the help of PerlPrimer software v. 1.1.16 (Marshall, 2004 Bioinformatics 20:2471-2472). Sequence comparison and dotplot analysis were carried out using NCBI BLAST and Dotmatcher (EMBOSS Package v. 6.0.1). Gene structure prediction of *MAT1-1-1* was performed with FGENESH software (http://linux1.softberry.com) using *N. crassa* as a reference genome.

### 1.2. RNA isolation, RT-PCR and RACE analyses

Total RNA was isolated from ascocarps stored at -80 °C according to the protocol described by Chang et al. (1999) Plant Molecular Biology Reports 11: 113-116. Rapid amplification of cDNA ends (RACE) was carried out using the SMART-RACE cDNA Amplification Kit (Clontech, Palo Alto, CA, USA) according to the manufacturer's protocol. The 5' and 3' RACE gene-specific primers were p21 and p23, and the nested primers were p22 and p24, respectively (Table 1, Fig. 1). To confirm the structure of *MAT1-1-1* gene, about 5 µg of total RNA isolated from two ascocarps that had gleba with the idiomorph *MAT1-1* were reverse-transcribed using the SuperScript® III RT enzyme (Invitrogen, Carlsbad, CA) and 200 ng of random primers (Invitrogen, Carlsbad, CA) according to the instructions of the enzyme's supplier. First-strand cDNAs were then amplified according to the PCR protocol reported above, using the primers p27 and p28 (Table 1 Figure 1).

**Table 1 list of primers used**

| Primer name | Primer sequence 5'-3' | Primer name | Primer sequence 5'-3' |
|---|---|---|---|
| p1 | CAGGTCCGTCATCTCCTTCCAGCAG (SEQ ID N°7) | p15 | TATCGTACACCACTTTCCTTCTATCTG |
| p2 | CCACATGCGACCGAGAATCTTGGCTA (SEQ ID N°8) | p16 | GATCTTCAGCCACTGGCGAATATCC |
| p3 | CAATCTCTTCCATCGCCCGTCCAG | p17 | GTTCCTCCGATCTTCCAAGAATTCCAG |
| p4 | TGGTATATGTGGATGTATTGATAACTATAAT (SEQ ID N°11) | P18 | TAATTACATTCCCAGAGCTAGGTGCCC (SEQ ID N°14) |
| p5 | AGAGATAGAGAAATAGCATGGCTCGG (SEQ ID N°12) | P19 | CAATCTCACTCGTGATGTCTGGGTC (SEQ ID N°9) |
| p6 | AAGTAACCTTTGTGCCATTGCTCCA | p20 | TCTCGGGCTGGAGGTGCGGGTCGAGT (SEQ ID N°10) |
| p7 | CAGCATGGTCTGGTTGGACGTTTTGAT | p21 | GCCGGAATAGTGGCATCCTGGGCCGTC |
| p8 | CTATGCACCCAAGTGTGATTGAGGAC | p22 | GACGGACGGTTGATTGAATTGCACCGAG |
| p9 | CCATTCGTTGAGATGCGCCGTTGCTA | p23 | GATCGTGGTGACCCCGGCCTCGTGCT |
| p10 | AGGCGGTATCAGAGCTTTTCCATGCAG | p24 | CTTGCGTGAAGCTGGCTTTGTCCCCAAG |
| p11 | GCCAACCTCTAGTTGGGATATTTGTTCAGGAC | p25 | GTGTGCGTGCTTGAAGTGAATTGGAGTAA (SEQ ID N°13) |
| p12 | GTGAATAGACAGACTAGCTTGCCCG | p26 | ATCCTAACTTACAGCATCTCGCCT |
| p13 | AATCTCTTAATGTCCAGACCCTTACC | p27 | TATCGGCTCACAATCCAGTGGAGGATTC |
| p14 | TGAATCAAGGTAGTTTGCTGATTGAGG | P28 | ACGGCCCAGGATGCCACTATTC |

### 2. Results

### 2.1. Searching for mating type genes in T. melanosporum genome.

The *T*. *melanosporum* genome sequence database "TuberDB" (http://mycor.nancy.inra.fr/IMGC/TuberGenome/) was investigated for the presence of orthologs of Ascomycetes *MAT* genes in the sequenced strain Mel28 using BLASTN and TBLASTX similarity searches. Orthologs, are homologous genes in different species that are similar to each other because they originated from a common ancestor. Using Ascomycetes HMG-containing sequences as queries, 3 candidate gene models were identified in the Mel28 genome: GSTUMT00001090001 (scaffold 247), GSTUMT00000587001 (scaffold 207) and GSTUMT00008644001 (scaffold 49). BLASTP searches against GenBank revealed that the GSTUMT00001090001 predicted protein was the best match for the *MAT1-2-1* gene from *Diaporthe* sp. (BAE93753, BAE93759), *Cryphonectria parasitica* (AAK83343), *Fusarium sacchari* (BAE94382), and other Ascomycetes. The GSTUMT00000587001 predicted protein showed the best BLASTP match with HMG-box containing proteins, especially with a Ste11 transcription factor of *S*. *pombe* (CAA77507), whereas the GSTUMT00008644001 predicted protein showed similarity with the HMG-box transcriptional regulator of A. *fumigatus* (XP_751745.1). Conversely, BLAST searches using α-box- and homeodomain-containing *MAT* genes from different Ascomycetes as queries did not allow the identification of any significant matches with putative sex genes in the Mel28 genome.

### 2.2. Selection of gene model harbouring the MAT1-2-1 gene and identification of strains harbouring the opposite mating type (figures 1, 2a, 2b and 2c).

To assess for the presence and distribution of the three HMG-box-containing genes in different *T. melanosporum* strains, the primers pairs: p1/p2, p7/p8, and p9/p10, specific to the gene models GSTUMT00001090001, GSTUMT00000587001 and GSTUMT00008644001, respectively, where used to perform a PCR screening on 12 *T*. *melanosporum* ascocarps of different geographical origin.

The GSTUMT00000587001 and GSTUMT00008644001 gene-specific amplicons were obtained from all the ascocarps tested, figures 2a and 2b. Conversely, only ten of the twelve samples screened using the primer pair p1/p2 produced the expected amplicon of 550 bp, figure 2c.

On the basis of these results the GSTUMT00001090001 was selected as the sex- candidate MAT1-2-1 gene and the two samples (me206 and me151) that did not produce the GSTUMT00001090001-specific amplicon regarded to as strains putatively harbouring the opposite mating type idiomorph (*MAT1-1*)*.*

The couple of primers p1/p2 can be used to amplify a nucleotide sequence specific to the first mating type gene of *T. melanosporum* (MAT1-2-1). The primer p1 has, as nucleotide sequence, the nucleotide sequence SEQ ID N°7 and the primer p2 has, as nucleotide sequence, the nucleotide sequence SEQ ID N°8.

### 2.3. Isolation of MAT1-1 idiomorph (figure 1, table 1).

Embracing the hypothesis of heterothallism, the 5' and 3' regions flanking the two putative idiomorphic structures are expected to be conserved between the sequenced strain (Mel28) and strains that lack the *MAT1-2-1* genes such as those that form the gleba of truffles me206 and me151.

Thus, on Mel28 strain, a set of primers were designed upstream and downstream the *MAT1-2-1* gene to search for genomic regions conserved between strains of different mating type (Table 1, figures 2a,2b,2c). To this purpose, on the 5' MAT1-2-1 flanking region, the primer pairs p3/p12, p13/p14, p15/p14 and p3/p14 (Table 1, Fig. 1) were tested for their capacity to yield amplicons of expected length of about 550, 400, 1700 and 2800 bp, respectively, on all samples screened. These primers pairs produced amplicons only when strains containing the *MAT1-2* idiomorph were amplified. Conversely, the primer pair p3/p4 yielded an amplicon of about 370 bp from both *MAT1-2* and *MAT1-1* strains. On the 3' *MAT1-2-1* flanking region of both the *MAT1-2* and *MAT1-1* strains, the primer pairs p16/p6 and p17/p18 yielded amplicons of about 400 and 1000 bp, respectively. The primer pair p5/p6 produced an amplification product of about 1200 bp in *MAT1-2* strains and a fragment of about 1700 bp in MAT1-1 strains. Overall, these preliminary screenings show that the binding capacity of p3, p4 and of p5, p6 primers was conserved in the upstream and downstream *MAT1-2-1* gene regions, respectively, in strains both with and without the *MAT1-2* idiomorph.

The PCR fragments obtained from sample me206 with primer pairs p3/p4 and p5/p6 were cloned and sequenced. The alignments showed that that these two fragments were highly similar (about 96% of sequence identity) to the corresponding regions in the Me128 genome, although the fragment generated by the primer pair p5/p6 (figure 1, fragment Fc) showed an insertion of 493 bp in the 3' region downstream the MAT1-1 idiomorph (figure 1 b).

On this insertion, the primer p11, specific for *MAT1-1* strains, was designed and used in combination with the primer p3 to amplify the genomic region containing the *MAT1-1* idiomorph from strain me206.

The amplification with the primer p11 and the primer p3 yielded a fragment of about 9 Kbp (figure 1, fragment Fa) whose sequencing confirmed the identification of the *MAT1-1* idiomorph. Indeed, BLASTX search against GenBank revealed the presence of about 200 bp region with similarity to the α-box domain that typifies the *MAT*1-1-1 gene of filamentous Ascomycetes. In particular, BLASTX matches were observed with α-box domains of MAT1-1 proteins of *Alternaria brassicae* (AAK85543.1), *Talaromyces stipitatus* (XP_002486084), *Aspergillus nidulans* (XP_660359), *Pyrenophora sp.* (CAP08782), and *Penicillium marneffei* (ABC68484).

The genomic fragment containing the *MAT1-1* idiomorph was further extended by PCR at the 5' and 3' ends using the primers p24 and p26 coupled with the MAT1-2 primers p25 and p18, respectively. The sequenced fragments Fa, Fb, Fc, and Fd were assembled in a 15,144 bp contig (figure 1).

The couple of p19/p20 was then designated and can be used to amplify a nucleotide sequence specific to the second mating type gene of *T*. *melanosporum* (MAT1-1-1). The primer p19 has, as nucleotide sequence, the nucleotide sequence SEQ ID N°9. The primer p20 has, as nucleotide sequence, the nucleotide sequence SEQ ID N°10.

Mating type specific amplification can be also obtained using any others couples of primers that are specific to the first and/or to the second mating type idiomorph of *T.melanosporum.*

The entire *MAT1-2* idiomorph sequence or SEQ ID N°3 nucleotide sequence is available in *T. melanosporum* genome database "TuberDB" (http://mycor.nancy.inra.fr/IMGC/TuberGenome/).

The entire MAT1-1 idiomorph sequence or SEQ ID N°4 was deposited in GenBank under the accession number HM370280.

### 2.4. Structure of MAT idiomorphs.

Sequence alignment and dotplot analyses of the two genomic regions showed that the *MAT1-2* and *MAT1-1* idiomorphs were about 5550 bp and 7430 bp long, respectively (A in figure 1a, B in figure 1b and figure 3). The MAT1-2 idiomorph has as nucleotide sequence the nucleotide sequence SEQ ID N°3 and the MAT1-1 idiomorph has as nucleotide sequence the nucleotide sequence SEQ ID N°4. The sequences flanking these idiomorphs were highly conserved: pairwise sequence alignment showed that the 5' flanking regions (3603 bp) share 99,1% sequence identity and the 3' flanking regions (3586 bp), excluding the 493-bp long insertion of MAT1-1, share 91,3% of identity.

Each idiomorph contains only a single *MAT* gene (HMG or α-box containing gene).

However, blast and dotplot analyses (figures 4 and 3) revealed that the idiomorphic regions were not completely different. In fact, the presence of shared sequences arranged in inverse orientations was observed (figure 1). Pairwise alignments showed 960 bp- (figure 1: G box) and 950 bp- (figure 1: H box) long sequences sharing 76.5% and 71.1% sequence identity, respectively. Additionally, BLASTX analysis revealed a region within the 960-bp sequence (G box) with similarity to conserved ankyrin repeat domains. An EST sequence (SY0AAA61YO16FM1) from Mel28 mycelium relative to GSTUMT00001089001 proved that the transcript of this gene extends its 3' end into the ankyrin-containing region, but that this last region is not included in the predicted CDS because it is only 57 amino acids long. Furthermore, no ORF could be identified in the corresponding region of the MAT1-1 idiomorph.

Finally, a short repeated sequence of about 460 bp was present in inverse orientation in the *MAT1-2* idiomorph (figure. 1: F boxes). A similar sequence (84% sequence identity) was also present at the extreme 3' end of the MAT1-1 idiomorph.

### 2.5. Structure of MAT genes.

The analysis of EST produced by the *Tuber* Genome Consortium showed that the *T. melanosporum MAT1-2-1* gene (*TmelMAT1-2-1*) consisted of 4 exons since it showed 2 additional introns to the one conserved in most of the Ascomycetes (figures 1a and 4a). The deduced amino acid sequence was 297 residues long and contained a conserved HMG-box motif typical of the *MAT1-2-1* genes of Ascomycetes (figure 4a).

Gene prediction analysis (performed with FGENESH in the *MAT1-1* idiomorph) suggested the presence of 3 exons within the *T. melanosporum MAT 1-1-1* gene *(TmelMAT1-1-1),* figure 1b. RACE and RT-PCR analyses were performed on RNA isolated from fruiting bodies harbouring the *MAT1-1* idiomorph to confirm the predicted gene structure. Interestingly, these analyses showed that the second intron could be differentially spliced to give putative proteins of either 319 or 399 aminoacids, (figures 5a and 5b). When RACE analysis was performed using the polyA-anchored primer in combination with the primers for 5' RACE (p21, p22) on the 5' extended cDNA pools as control, it revealed the presence of an antisense transcript partially overlapping the MAT1-1-1 CDS (figures 5a and 5b).

### 2.6. MAT genes are expressed both during the vegetative and reproductive phases.

RT-PCR analyses along with the availability of ESTs (http://mycor.nancy.inra.fr/) from fruit body (FB) free living mycelia (FLM) and ectomycorrhizae (ECM) (Martin et al. 2010, Nature 464:1033-1038) allowed the inventors to evaluate transcription of MAT genes at different phases of the *T. melanosporum* life cycle.

The analysis of EST datasets confirmed that in FLM of the Mel28 strain used for genome sequencing, only the *MAT1-2-1* gene was transcribed. On the contrary, both *MAT1-1-1* and *MAT1-2-1* transcripts were detected in the EST libraries obtained from FB and ECMs.

### 2.7. MAT idiomorphs are equally distributed among truffles within single populations.

The presence and distribution of mating type genes in the ascomata collected from natural populations were assessed using a multiplex-PCR approach. To this end, the α-box-specific primer pair p19 (SEQ ID N°9) / p20 (SEQ ID N°10) was designed to produce an amplicon of 421 bp and combined with the HMG box-specific primer pair p1 (SEQ ID N°7) / p2 (SEQ ID N°8) that produced an amplicon of 550 bp. As shown in Table 2, truffles harbouring either the *MAT1-2* or the *MAT1-1* idiomorphs were identified within each local population.

### 2.8. All T. melanosporum truffles results from outcrossing.

To test the hypothesis that all *T. melanosporum* truffles were derived from outcrossing and that *T. melanosporum* is a "truly" heterothallic spp., the multiplex PCR approach described above was carried out on asci and gleba isolated from single, selected ascocarps.

When screened with SSR and single nucleotide polymorphism (SNP) markers, the asci of most ascocarps did not display any additional alleles with respect to their corresponding gleba (Riccioni et al., 2008, New Phytologist 180: 466-478). Whether these truffles resulted from the crossing of two parents or just from selfing of homothallic strains remained elusive. Multiplex PCR-based mating type screening showed that the gleba of these ascomata had either the MAT1-2- or MAT1-1-specific allele (figure 6a). In stark contrast, the pool of spores always presented, with an approximately equal intensity, of both MAT1-2- and MAT1-1-specific amplicons, irrespective of the truffle samples processed, the mating type of the corresponding gleba, and the number of PCR cycles performed (figure 6b).

Altogether, these results suggest that the additional mating idiomorph detected in the asci with respect to the surrounding gleba must be contributed by a partner with a different sexual polarity, and that this condition is common to all truffles screened.

**Table 2 : List of truffles whose gleba was genotyped using mating-specific primers**

| **Sample** | **Population** | **Mating type** |
|---|---|---|
| 442 | 2 | α-box |
| 443 | 2 | hmg |
| 444 | 2 | α-box |
| 445 | 2 | α-box |
| 446 | 2 | α-box |
| 448 | 2 | hmg |
| 458 | 2 | hmg |
| 471 | 2 | α-box |
| 478 | 2 | α-box |
| 459 | 2 | hmg |
| 476 | 2 | α-box |
| 482 | 2 | hmg |
| 280 | 2 | α-box |
| 284/287 | 2 | hmg |
| 281 | 2 | α-box |
| 206 | 2 | α-box |
| 151 | 2 | α-box |
| 66 | 6 | hmg |
| 69 | 6 | α-box |
| 67 | 6 | hmg |
| 68 | 6 | hmg |
| 71 | 6 | α-box |
| 70 | 6 | hmg |
| 296 | 7 | hmg |
| 300/303 | 7 | hmg |
| 298 | 7 | α-box |
| 180 | 10 | α-box |
| 224 | 10 | hmg |
| 222/182/226/334/335/227 | 10 | α-box |
| 225 | 10 | hmg |
| 336 | 10 | α-box |
| 337 | 10 | hmg |
| 183 | 10 | α-box |
| 294 | 12 | hmg |
| 367 | 12 | α-box |
| 81 | 12 | hmg |
| 83 | 12 | hmg |

## Claims

1. A method for determining the *Tuber* species of a truffle sample, said method comprising identifying whether said truffle sample comprises at least one nucleotide sequence chosen among a first nucleotide sequence associated with a first mating type idiomorph (MAT1-2) and a second nucleotide sequence associated with a second mating type idiomorph (MAT1-1); both nucleotide sequence being specific to a given *Tuber* species.

2. The method of claim 1, further comprising determining the fertility of said truffle sample, using the identification in said truffle sample of both the first nucleotide sequence associated with the first mating type idiomorph (MAT1-2) and the second nucleotide sequence associated with a second mating type idiomorph (MAT1-1), the fertility of said truffle sample requiring the presence of both the first and the second nucleotide sequences.

3. The method of one of the claims 1 to 2, wherein the first nucleotide sequence comprises the nucleotide sequence SEQ ID N°5 and the second nucleotide sequence comprises the nucleotide sequence SEQ ID N°6.

4. The method of one of the claims 1 to 3, using a first pair of oligonucleotide primers to determine the presence of the first mating type idiomorph (MAT1-2) in said truffle sample, each primer of the first pair of oligonucleotide primers comprising from 5 to 50, and preferentially from 10 to 30 successive nucleotides of the first nucleotide sequence.

5. The method of claim 4, wherein a forward primer of the first pair of oligonucleotide primers comprises the nucleotide sequence SEQ ID N°7.

6. The method of one of the claims 4 to 5, wherein a reverse primer of the first pair of oligonucleotide primers comprises the nucleotide sequence SEQ ID N°8.

7. The method of one of the claims 1 to 6, using a second pair of oligonucleotide primers to determine the presence of the second mating type idiomorph (MAT1-1) in said truffle sample, each primer of the second pair of oligonucleotide primers comprising from 5 to 50, and preferentially from 10 to 30 successive nucleotides of the second nucleotide sequence.

8. The method of claim 7, wherein a forward primer of the second pair of oligonucleotide primers comprises the nucleotide sequence SEQ ID N°9.

9. The method of one of the claims 7 to 8, wherein a reverse primer of the second pair of oligonucleotide primers comprises the nucleotide sequence SEQ ID N°10.

10. The method of one of the claims 3 to 9, wherein each of the nucleotide sequences SEQ ID N°5 and SEQ ID N°6 are specific to *Tuber melanosporum.*

11. Nucleotide sequence associated with a first mating type idiomorph (MAT1-2) of a *Tuber* species comprising the nucleotide sequence SEQ ID N°5.

12. Nucleotide sequence associated with a second mating type idiomorph (MAT1-1) of a *Tuber* species comprising the nucleotide sequence SEQ ID N°6.

13. Pair of oligonucleotide primers associated with a first mating type idiomorph (MAT1-2-1) of a *Tuber* species, wherein each primer comprises from 5 to 50, for preference from 10 to 30 successive nucleotides of the nucleotide sequence SEQ ID N°5.

14. Pair of oligonucleotide primers associated with a second mating type idiomorph (MAT1-1-1) of a *Tuber* species, wherein each primer comprises from 5 to 50, for preference from 10 to 30 successive nucleotides of the nucleotide sequence SEQ ID N°6.

15. Kit for determining the fertility of a truffle sample, like truffle fructification sample, truffle mycelium sample, mycorrhized plant sample or truffle-producing soil sample, the kit comprising:
- the pair of oligonucleotide primers, according to claim 13, and
- the pair of oligonucleotide primers, according to claim 14.


SEQUENCE LISTING

<110>  INSTITUT NATIONAL DE LA RECHERCHE AGRONOMIQUE

<120>  MOLECULAR METHOD FOR THE IDENTIFICATION OF MATING TYPE GENES OF
       TRUFFLES SPECIES

<130>  24416EP

<160>  14

<170>  PatentIn version 3.3

<210>  1
<211>  1062
<212>  DNA
<213>  Tuber melanosporum

<400>  1
atgactgctt tgtccatgct caatatggcc gttgaagccg ggcctcttat tcaccaacgt      60

tatgatggga tcggtctcgc cgagtatatt cccttgctcg ccaatggaac tttttctttt     120

gatgggtcgg atggagcccc gctccaggtc cgtcatctcc ttccagcaga ctggaagccc     180

gtcaatggac cacttacggt cttgttcgat tccgcccccc ataccattat tgggactcac     240

ttctctatcc acaatcatcc catttacaag cttaacgatg gttctatctt tgttgtcttg     300

gacctggcca ccaagcacgg ttttgagttt accaatcaat ctttcatgga cccccagctt     360

gaggtctccg gggaaaaccg tttcttgcgt ctcgttacca atgaccaaga cttcgcctac     420

catggctcta gtcttagcat gattcatgac aagggcgtta agcgccctgc caatgcgtaa     480

gtacaattct accaatgtac cagtttcgtg tgctcatata gtcataggtt tatcttgttc     540

agaacggaca aaatgaagcc cctcaaggct cagtacccgg agatgggcaa caacgatatc     600

tgtacgtggc tctcattttc cctttattta gatctctatt cttgctacca tttgcctaac     660

gttttaatag ccaagattct cggtcgcatg tggcagaatt cctcggatga ggtcaaaaat     720

gtttataagg taaaacatct cacctaactg ttagttagaa tcactaacac attaataggc     780

aagggcccat cagcttgcgg tttcccacaa gttgatcaac ccggattaca aatactcccc     840

gcgccgctct cgcgaaatca tgaggagggg ctccgggaat aaaggtcctt cggcccgtat     900

cgaccttcat cgtgctggtg ttgcgaagcg tggaaaaaag aggatcatta cccaccttgg     960

gttcctcact caacgtttcg cgactcaccc cactgctaac aatctcggtg atatcatcga    1020

ggaagccgaa aaggtcttga aggcctttgg tatggtcaat tg                       1062


<210>  2
<211>  1069
<212>  DNA
<213>  Tuber melanosporum

<400>  2
atgcgtaatg ttaccttggg atgcgccttg tacctttcgg accgtgggta cattctcatg      60

caagacagga tcgggtgtat ctgggtccag cacgagaact cccccattat cgttcaaccc     120

cccggtggtg tcactttcac tgtcagcggc gaactcttgc cctacgacaa gtggctcggt     180

gcaattcaat caaccgtccg tcatcaaggg cgagtccccc tcgaccccga agtcccttat 240

gttgtgtcgg ttctgcgttt gtattgcgga tactataaac ctggacagcc gtggacggcc 300

caggatgcca ctattccggc tgttatcgtc aatggtgatg aggccttggc ccgtattccc 360

ccctctcagc accttcgtgc attgaatccc tacgttgcgc agagatgtaa gtaaatcaca 420

tccctatttt gcctctattt ctaaccatct attagcctgg atttctaagt actgtaacgg 480

ttatgggctc actcaagccg aaatttccaa tctcactcgt gatgtctggg tcgctgagac 540

caataagtat atgtggcaaa acatcgcctc tctttatact gctgctaggg atcgtggtga 600

ccccggcctc gtgctcgaag aatttatcga gaccgaattg gcgaaacacg gtcaccctac 660

tacccctgag aagctcttgc gtgaagctgg ctttgtcccc aaggccccca tcgacaagga 720

agcggccgat aaggctgctg caagaaggaa ggttcgtgcc aagcccgcac ccggggtgtc 780

cagtttcact atcacccgtg tttacgtttc cagcaaccgt cagctcgatc tcaccgagga 840

ggaagccgcc aacatcctcc atgctttcga catcgctagc gctgaggaga atggagactc 900

tcaactcgac ccgcacctcc agcccgagat cgagatgcac atgaacactt tcacttctgg 960

tggatacagc gtttttgtga gaagttcccc gaacagcata ttccgccact gcccaattct 1020

aacattatct aaacagccta ctattggaga atcctccact ggattgtga 1069

<210> 3
<211> 5486
<212> DNA
<213> Tuber melanosporum

<400> 3
tgagcgattg cttgcctgca ggcaagtgat ccggacccac cactaggtgg gcccggtacg 60

gggagcactg tctgtctgtt cgcttcactt gtgcggttcg aatgctttac ctggttgtgc 120

gctctgagta cgagaaatgg gcgggcaagc tagtctgtct attcacgaag ggcgcaatga 180

actatcacaa accaatcccg gattcttcta ctcttttgtc cctttctcac ggaggttctt 240

tccagtgagc ttcgcccggt ctcgtctcgt ccgccattgc gtggttattt acagcggatg 300

gcttaatccg cacccttgtc tatcatcgca atacggcatt ggctctggcg aaactatatc 360

gtgtcgttcc gagggagaaa aaaaaatccc ttcattctac tttcccttcg tccactccga 420

ttcactggca gatgaaaccg taccgcgcgc tcgggctctt tggtccctat tttctcaacc 480

ggcgagggcg tcgttagtat gggggttgga aggctccttt ggcttgtttg tcgtgagtag 540

ggagggcgct gtcattgctg agtagccttt cagcggggcc tgcgggggtg cttctttccg 600

atcggtcttg cgaggggtgg tattgctatt gggcggtgtt ggccattcgg cggcgttgat 660

gaaatttcta tcgggggttc tgtggcctct ggatacgtgg tgtcgtatat cttctggcgg 720

tttctctgct gctaagtatg atatcgcact attcgtgggg acctgtcatt aagtctttac 780

atagttgctc actatggtct taggaatcct tgcgctatcg tacaccactt tccttctatc 840

tgaacaacac tctatatgtt tatctatcgt acagggggag aaaggtaacg cgtgggcaag 900

aatgcacggc tggagaaaat aaaatatgat aggatgaaaa ccgcaaggta taataaacta 960

gacttttttg ccacagcgac tagttccaaa ctctacagta ctcagggggc aaacccacgc 1020
ccgactataa aagcaaatct ccaggggggc tcgcatcagc acggtgacat aagcgcattc 1080
cgggctcgtc tcatgggatc tccttcgact ggaaggggtg atgcctaagg acgactttcg 1140
tgggtagggt accccggaaa agaaggaacg gtgatgcctt cgatgaggtc ctgttttata 1200
agccttaaat cgcgctaggg gttaatgctt tgtgggactg tagcagcgct accaccttct 1260
gatggccgtg cctagatgcg aacgagagag gtgtttgacc gtcattgttt ggcctgtcgg 1320
ggctgacatc ttccccgccg agtagtacct tcaccacctc cgcatgccca tcccaagcag 1380
cgagcgagag cggtgtttgg ccgcaatcat ctggcctgtc agggttgacc tcttccctca 1440
tgagtaacat tttcaccact tcttcatgtc catgaaaggc agcatgagag agaggtgatt 1500
ggccgtggct gtctggcttg tcgggattga cctcttcccg catgagtagc attctcacca 1560
cctcttcata tccggaccag acggcatgcg agagcggtgt tcggcaatag atatctggct 1620
tgtcagggtt gacttcttcc cgcccgagaa agattttcac tactccctca tgtccagaac 1680
cagcggcatg cgagagtggc gttcggccga gattgtctgg cttgtcgggg ttgacctctt 1740
cccgctcgag taataatttc gcaactccct cataccccct ccaagcagca ttcgagagcg 1800
gtgtttggcc gtcattatct ggctcgttgg gattaacatc ctcccgcccg agcagtattc 1860
tcaccacttc ctcatgtcca aaccaagcag cgagcgagag cggtgtttgg ccgtcattgt 1920
ccggcttgtc ggggttgacc tcttcccgcc cgagcagtat tttcaccact tcctcatgtc 1980
cacaggcagt agcatgtgag agcggtgtct aggccttctc cgttcgttat attactatac 2040
catcctactg atctcagcat ggcctaactt agccaagaac ccctctgaaa ccaccaggag 2100
taacctctag caatgtcagc cacaatctct taatgtccag acccttaccg agaccggttg 2160
tcgcattgga tgactgcacc tttgttctgg agttccatct tttaaacagc tttcccgaga 2220
gctgcatacg ccacatcaaa cgtcgtagtc ctccttgtcc attcacctgg atgcacaagc 2280
ggatagccga acaggggaca ttcattggat cggctggtag tgtttccaag gcatttggct 2340
ttcctggttg gccgggcagt gggtgaagct accatacttc tgaattcaca tttcgcctaa 2400
cggcctcctg gccattggat atccccccag gacggaaaga acggcttctg gatggccagg 2460
attaaggtat ggtttcccag tccgccacac agatttatgg agatcatatt aaattcctca 2520
atcagcaaac taccttgatt cacatatctg atcacaaaga attattgttc agggctcgga 2580
taccatacct ctctatcaaa gaagcaatgt acaagagagg tggggttgga ggacggatgc 2640
tgtaacagaa cccacatttc ccagctcacc ctgtttcatt aattcctttg ttcggaactt 2700
ctccgacagt aaaaaaaaat tcgcggatct tttcgccagg aatcttggga acctaagggc 2760
tctctcttcc tgccctgggg gaaatcttat tgtgtcataa tattattatg tgtgataata 2820
ttatgacatt ttcaagatac cggtaatact attctcgggc cctagtaggg ttgagttgta 2880
cgtgacggct tcttggcgtc actctatttt ttgggtcttg gcatttcccc tcgtgacagg 2940
aggcccacta gggggatcca tcctttcctc cttctttcct ccgccgacaa ccgtcaagct 3000

ccaccgacaa ccgtcaagtt ccaccgacaa ccgtcaagtt cgacagaaac attcggcctt 3060
tctacttgca cctctccact cacgtcgttt ctttttttct gccactatga ctgctttgtc 3120
catgctcaat atggccgttg aagccgggcc tcttattcac caacgttatg atgggatcgg 3180
tctcgccgag tatattccct tgctcgccaa tggaactttt ttctttgatg ggtcggatgg 3240
agccccgctc caggtccgtc atctccttcc agcagactgg aagcccgtca atggaccact 3300
tacggtcttg ttcgattccg ccccccatac cattattggg actcacttct ctatccacaa 3360
tcatcccatt tacaagctta acgatggttc tatctttgtt gtcttggacc tggccaccaa 3420
gcacggtttt gagtttacca atcaatcttt catggacccc cagcttgagg tctccgggga 3480
aaaccgtttc ttgcgtctcg ttaccaatga ccaagacttc gcctaccatg gctctagtct 3540
tagcatgatt catgacaagg gcgttaagcg ccctgccaat gcgtaagtac aattctacca 3600
atgtaccagt ttcgtgtgct catatagtca taggtttatc ttgttcagaa cggacaaaat 3660
gaagcccctc aaggctcagt acccggagat gggcaacaac gatatctgta cgtggctctc 3720
attttccctt tatttagatc tctattcttg ctaccatttg cctaacgttt taatagccaa 3780
gattctcggt cgcatgtggc agaattcctc ggatgaggtc aaaaatgttt ataaggtaaa 3840
acatctcacc taactgttag ttagaatcac taacacatta ataggcaagg gcccatcagc 3900
ttgcggtttc ccacaagttg atcaacccgg attacaaata ctccccgcgc cgctctcgcg 3960
aaatcatgag gaggggctcc gggaataaag gtccttcggc ccgtatcgac cttcatcgtg 4020
ctggtgttgc gaagcgtgga aaaaagagga tcattaccca ccttgggttc ctcactcaac 4080
gtttcgcgac tcaccccact gctaacaatc tcggtgatat catcgaggaa gccgaaaagg 4140
tcttgaaggc ctttggtatg gtcaattgag tgatctctac ggagggactc atcggtacct 4200
gggtggtata cttttaatgg gagggatgat cttgtctatc aaaggtcatt cattatctca 4260
gtcgactaaa tttatgacct aaatagataa aaaatatctt ttaaaaccaa aatgaggggt 4320
ccgaacacta catcatacag taaatatcaa gttgtgaagc ggaagagagc cgactatggg 4380
aagaggcagt aagaatttaa ttctgactaa gtctggaaag tttacacagc acttgcacag 4440
aagttatgct atggtggtgc catcaccgga aaaacacgcc aaaaattcgg gacctatggt 4500
gccgtgactt agtttgagcg caagtacatg gttcgacaca aaagagtgtt acggagtagg 4560
ggtgttgggg ttgattttgg ccattagcgt tatcgtatag attgtttgaa aacacgactc 4620
gtattgctta acaagaatat ggagagagct atttttttaa ttttttttaa ttttgataga 4680
tgtattggac tggaggccaa gcatctgatc aattggttat actatactcc tatttgttca 4740
tagttgactg aggccctgtt tggctacaac ggttcctttc tgtttcccca tacaaagcga 4800
ccgttgcaga acgggagggc aatagctttt atatgagaaa cggttccaaa atgaaacgga 4860
accgaggaaa ttgtgatcta gtttcttgga tgtgccgctg ctactgaaat ttttgttata 4920
aaggtatccg ttttatatct gactcccttc acacagaccc caatggcgcg aatccccgga 4980
gtactttgct catgaaagtc gtcctgtcgt ccttgggcat caccccttcc agtcgaagga 5040

gatcccatga gacgagcccg gaatgcactt atatgtcacc atatggacat gaacctccgt 5100

tgagatttgc ttttacagtc gggcgttgtt ttatttcccg agtactttag agtttggaac 5160

cagttctatt gctttgacaa agtctagttt attatacctt gcagttttca tcttatcaca 5220

ttttattttc tctagccgta cattcttgcc ctcgcgtttt tttctcttgc acaactgata 5280

gacatataag tgttcttcag atagaaggaa atggcgtacg atagcgcaag gattcctaag 5340

accattgtga gaaactatgt aaagacttct gacaggtccc cacgaatagt gcaatatcat 5400

acttagcagc agagaaacca tcaagaaggt agactacagc acatagtcag aggccacgga 5460

actcctgaga cataagggta gggata 5486

<210> 4
<211> 7509
<212> DNA
<213> Tuber melanosporum

<400> 4
ggcttgcatg atatcagcga atgtagctcg aggacgtaaa tctgatagtt acactaaacg 60

ttggatacca cgctggttca gagctagctc cttctccgct cgaggtagag gattaagttc 120

accgccctct gaatggcatc tgatgatgtt ccgggcaact tctctcgggg cacctgtgtc 180

gctagcaatt gtatgataac ttttgccttt ctgcatgaag ctcaatcatt cgwactctct 240

catcgcttga ggccattcgc cgcttgcctg tttcttgacc ttgaggtatg gtgtatgatg 300

ggaggtgtta gctcctataa ggaaatcgtg gccaagatac tagtgtcggg ttactaaaat 360

aacctctgct gccgagaccg ttgcgaaagc tgggtagatt ctctctacat attctagtag 420

atcttgacgc tgatttacgg atggataaaa aggaatttta tgtttttttt ggttttaaag 480

ataccgaggg cgtttcattg atcctagata cataagttta atttaagtct acacgatcta 540

taatacgctg agaatgcctc ccaactatgc ccttgattcc cgccgctccc acacggtcat 600

accgtaatac agtaacttca tttttttttt gtacccacta gcctctttag gccttcatgg 660

gaggtcatat cggctcacaa tccagtggag gattctccaa tagtaggctg tttagataat 720

gttagaattg ggcagtggcg gaatatgctg ttcggggaac ttctcacaaa aacgctgtat 780

ccaccagaag tgaaagtgtt catgtgcatc tcgatctcgg gctggaggtg cgggtcgagt 840

tgagagtctc cattctcctc agcgctagcg atgtcgaaag catggaggat gttggcggct 900

tcctcctcgg tgagatcgag ctgacggttg ctggaaacgt aaacacgggt gatagtgaaa 960

ctggacaccc cgggtgcggg cttggcacga accttccttc ttgcagcagc cttatcggcc 1020

gcttccttgt cgatgggggc cttggggaca aagccagctt cacgcaagag cttctcaggg 1080

gtagtagggt gaccgtgttt cgccaattcg gtctcgataa attcttcgag cacgaggccg 1140

gggtcaccac gatccctagc agcagtataa agagaggcga tgttttgcca catatactta 1200

ttggtctcag cgacccagac atcacgagtg agattggaaa tttcggcttg agtgagccca 1260

taaccgttac agtacttaga aatccaggct aatagatggt tagaaataga ggcaaaatag 1320

ggatgtgatt tacttacatc tctgcgcaac gtagggattc aatgcacgaa ggtgctgaga 1380
ggggggaata cgggccaagg cctcatcacc attgacgata acagccggaa tagtggcatc 1440
ctgggccgtc cacggctgtc caggtttata gtatccgcaa tacaaacgca gaaccgacac 1500
aacataaggg acttcggggt cgagggggac tcgcccttga tgacggacgg ttgattgaat 1560
tgcaccgagc cacttgtcgt agggcaagag ttcgccgctg acagtgaaag tgacaccacc 1620
ggggggttga acgataatgg gggagttctc gtgctggacc cagatacacc cgatcctgtc 1680
ttgcatgaga atgtacccac ggtccgaaag gtacaaggcg catcccaagg taacattacg 1740
cataatgagg cgtctctcga gcctgttctc ccactccgaa accttagggg ggggggagag 1800
gttaacgacc atgctcagat tgacaggacg agacgacatt atgaaaggtg ggggggcggt 1860
tggagacggg aaaagaaatt tggaccgaca aaaggaggat tgcgtatggg cttttcgatg 1920
agtggagatt ggcctcagtg gctgttggtg gtggaagaaa ggagggtgga aggtctggca 1980
acttttgaag gctacccgtc acgaagtggg atgccaaggc ccaaaaaaaa gagtgacgcc 2040
aagaagctgt cacgtacaac ttagccctac tagggtctat cataatacat actatacgat 2100
aataaattat tatgatatta tcataagatt ttccccagag caggtagaga gcccttaggt 2160
ttccaagatt cctggcggaa agatcgggga acatttttct ttttttacgg tcagagttgc 2220
tcgaatagaa aaatgagtca aggtatttga tgaagatctg atcgacattc gggagatgtt 2280
tagaacaaag gaatttagtg gggatgagct gggaaatgtg ggttctgtta cagtatctgt 2340
cccccaatct taactagccc tcttgtacat tgcctctttg gtaaaggggt atggtatctg 2400
agccttgaac agtaattcct tgcgatagga tatatcgatc aggatagttt gctggctgga 2460
aaatttaata tagtcttcat aaaatcgtgt gacggactgg gaaatcatac cttgagtttg 2520
gccatccgga agcctctctt tctgctatga gggaatatca aatagccaaa aggccatggg 2580
gtgaaatgag aatttagaaa tatgatagcg gtcccttgcc accaaaagac aaatgcttcg 2640
gcaataccat cacccgatcc ggtggatgtc tccatgttcg gctatccgct tgagcatcca 2700
ggggaataga caaggaagac taccacattt gatgcggtgt gtacggctgc cgagaaaact 2760
gtttgaaaga tggaactcca gaacaaagga ttaactggtc aacgcaatac ctggttgccg 2820
caagagtctg aagattaaag gattgtaact agtattgttg gaggttactt ctagcaattc 2880
tagaggggtt tttggtaaag atagattata ctgatatcag tcggatgata tggcactaca 2940
acgaaagaaa gtggtctaga ccatgagcgc aaaaactcca aaaagaattt cctgccggga 3000
aattagaata tttaaccggt ttaaaaccgt aagatttgct ggtccaggac cagcggcaaa 3060
ttgagagcca ttgtgagagc atctaagaac tcaaccttca gaactccagg ataagtagga 3120
tgataaaagg gaaatggggt ttagaatggg tgcataaagc cttttgattc tggattaaga 3180
tatggccaga taagggagtc tgaagcaatg tgcatttaat ctccgctcgg actcctagga 3240
taaaaacgga catgacaagt aaattataat gaggaaaaaa gcttgattgg ttgtaggggt 3300
cgattgtatg atcagaggtg ggggtactac aagtttatcg atataatatt gggaataact 3360

gtgaaaatgg ttccagaata tagctgagta ttttacaaac atgcattagt tgcatatgag 3420
atatcataga tactagagtc gccttgttca gtctcttggc tgtctttgaa tatctagata 3480
taccgggaaa gaaaatggga aagaaaatat catcggcttc tttagtaaac tcttcaaaga 3540
tagcaaaaac ccaacctcta cttacagcct gagcctatag cttggtatgg tactcattta 3600
ccctttcttc cacagagcta tgcacttgca atatttcttc caactgtttg tttaaaatcc 3660
atgaggttcg acatcaaatg aaaggggtaa tttccacatg cacaaaacat atattgtgtg 3720
aatacctaca attgagcctg cataaacaca tgaacccaaa tcaattcagt aaattaaaac 3780
ccatcaagta gttggtgatt tagctcatga aatttgtgaa gtttttttga gctaaaggga 3840
aaagagaaat gttacgcaaa tttttttgtc acccttagct ggtcggctgt caatgcttga 3900
tattgtgata taaattacta caggttgtat tgcgggaatg ttgacatatt gcagtacgac 3960
atcttctaag gccatatgca tataagagat tactttatcg tactaagaaa gccaagacaa 4020
tgatttctac tacttttttc tatattctct tacctcatat tccactatga tttacaagag 4080
tcatgccaaa tcaagtattg tgtgatacct ggtgaaaaat atccatcaaa gcctcagagc 4140
tgtaaatatc gcaaattgta aggtgagagg ggaatcacaa tgcataatat tatataaaca 4200
ccacaactgc tctactgggg gtgccttatg ctaggacttt cctacatgcg acccattaac 4260
agattttaaa aaagaaaaaa agtcactgag gatgccccac tgccacatta aaggagagag 4320
tagttcttag atcttgtctc cctcagcttg cttagtgcac ctgtggacag aaactcaggt 4380
gaagatagag aaagaggtat catgaatttt aatgcacagt attcattgca atttaattcc 4440
gggcacacta agttatttat tcatttactc aatccctact gcaatccaag ctactcttgt 4500
ccttgcccag gctatatctt gagggcgggg aacaaactta ttctcattgc accttctggg 4560
cgctacgagc gaaagattga ctatttcatc aattttaaat cctaaataca agtatcatac 4620
acatctactc aagtacaaga atccttgtaa cgattggaac aaaacaaatt ctgggactct 4680
tgtcggacac tacgttaacg ctagtaagta acaaaaaatc tttttaatat tagtagtgtg 4740
tgtgacatcc tcttagattt tgcgtgacac ccctgccgct actaggtctt agtactcgag 4800
tagtagaata ataggagtag cgaccttcaa cgacagccaa gcgatcaaaa agagtatcgt 4860
actcgagtat tcaaatggaa aatctacctt cctatgatac caggtgaggt cattgcggtg 4920
gttcaatacg acgatcaaat cctgcgatta cgcataacaa gtgtgagtgc tcgagaagag 4980
cgcgaatgca cagctgcctg gcctgcgtgg gtctgacaga tctgtccttg ctctatttat 5040
ttgttttgtt tttcctgagc gcagcacttt ttaccctatg aggtatttat aataatatta 5100
tggatattct ggaggaggtt ccaaaaaata tatcggaaat gcacttaggg gcaatggtac 5160
taggattcca tcgcacacta tccgctaata gatatatcta ggtttctagt ttccataaat 5220
atcagacgag aaaggcttac caaaatgacc aagggggttg ggtctgggag atgtctacgg 5280
tgcgacgata gaacggataa aagcgcaggg cggggataaa tcgagactcg ggatggcagc 5340
tttgatgtgg atcagtcatg cgcagcggct actcgggatg gacgagctct cccacgccct 5400

ggcagttgag ctgaactcca ccgacttcaa cgctagcaac atctcttcga tgtcgacact 5460
ggtgggttgc ttccaagggc ttattaaggt ggacaaggaa gcatcagccg tgcaactggc 5520
ccactttact ctccaagagt acctttccgc tcatcccgat attttctgca gacgccatgc 5580
ggcaatggca gagatctgct taacctatct gaattcccgg caaatcaagg acctttcgac 5640
cgaccatctc ctgacacccc ggatgcaccc tttctagaat actgttccgt gcactgggga 5700
atccatgcga aaacggaact ctcggatggc aggagatcgc ttgcgctgaa gctattctag 5760
gagtttgatg gccacatatc cacagaattt cttttagcgc aagtggagaa tttaagttcc 5820
caggggcgtg atacctgacc tccgtccagt gggttgtact gcgcatcgtt ctttggaatc 5880
gtcgaggttg tggctgcttt gacatccgtg ggatgttggg atgccaaaga agggggttgt 5940
ttggggtaac cgccacttgc acgggttcat aacgggtatg gggaggtggt ggaaatgcta 6000
ctcgggttgg aacaggtcca tcccgacgag ccggataacg cgaccggaca ccgctctcac 6060
atgttactgc ctgtgggcat gaagaagtgg tgaaaatact gctcctgttt gagcggggag 6120
aggctagcca cgaaaagccg gacaatgacg gtcaaacacc actctctttc gctgcttggt 6180
ttggacatgg ggaagtggtg agaatactac ttgggcggaa gatgttaatc ccaatgagcc 6240
agataatgac ggccaaacac cgctctcgaa tgctgcttgg aggggtcatg agagagttgc 6300
gaaaatatta ctcgggcggg aagaggtcaa cctcgaagag ccagacgagc tcggccgggc 6360
gccgctcttg catgccgctg gtcatggaca tgagggagta gtgaaaattt tctcgggcgg 6420
gaaggggtca acctcgaaaa gctagacaat tactcccgaa caccgctttt gcatgccgtt 6480
tggtctagat atgaagaggt ggtgagtgtg ctactcatag ggggggtgtc gatctcggcg 6540
aaccggacag cagcggccaa ataccgctct cgcatgctgc cgctcatggg cacgaggagg 6600
tggtgaaaat gttactcatg tgggaagagg tcaaacccga caggccagat gatttcggcc 6660
aaacactgcg ctcgcttgct gcttgggatg gacatggggg atgatgaaag tactactcgg 6720
ccgggaggat gtcaaaccgg acaggccaga cgatagcggc aaaaccctgc tctagttggc 6780
cgccgggatg ggcatgaggt agtggtgaaa atactactcg ggcgcgaagt tgtcagtcct 6840
gacaaaccag ataatggcga ccaaacaccc ctctcgttcg cagctgggaa tgaccatcag 6900
aaggtggtgg ccctgctaca gtcccacaaa gcgttaaccc ctagcgcggt ttaaggttta 6960
taaaaaacaa cctcttcgaa ggcatcacct tccttttctt ccagagtact ttgctcgatg 7020
ggggtcatcc tagggcatca tcctttccag tcgaaggaga tcccatgaga cgagcccgga 7080
atgcgcttat atgccaccat gcggatgtga acctccctgg ggatttgctt ttatagtcgg 7140
gcgtggattt ttttttctga gtacggtaga gtttggaact agttctatcg ccgtgacaaa 7200
gtttagttta gtttattaca ccttgcagtc ctcatcctat catatttgat ttcctccagc 7260
cgtacattct tgccctcacg ttttccctct cccttgcgca attgataaac atatagagtg 7320
ttcatataga aggaaagtgg tgtacgagag gggaagggtt cttaagacca atgtgagcaa 7380
ctatgtaagg actgaatgat aggtccccac gaatagtgcg atatcatact cagcagcaga 7440

gaaaccatca agaaggtaac tacagcacat atccagaggc cccagacctg tgacataagg 7500

atagggata 7509

<210> 5
<211> 12757
<212> DNA
<213> Tuber melanosporum

<400> 5
gtgtgcgtgc ttgaagtgaa ttggagtaaa gtgaaggtgg tggtggtgaa agtggcgaat 60

atgagagtat caaaggagga ggtgagcaga gcattgagct tattagtagg ttcagagcga 120

gttgcgtgcg ctgtctctag ggttagagtt acagttagga tgcggcgttg tgtgtgtgtg 180

ggcgtgggtg cgtgggtgga tatgtgggtg tgtgtgttat tcttatcatc tgagtttgtt 240

gtcgtctttt caatttcact cgagtcgctg taggaggcag gagggagagt tgtacgagta 300

ctgtagatgt agagcacctg tatcatatta tcatactaca cacagtacag tactgcacga 360

gcacgagcac gaatacggtt tcagtgaatc tggagcttca ctcgtttgtg ttttttagct 420

tcgtgcactt ttgtcaatag agtaattatt taatatcatg atggttatta taatatatta 480

tcatacaggt ggtggagctg tatgataccc ttcgtatcat atccttatgc atcacacatc 540

ctttgcataa caaagtagct ggttttaggc ctacagtgcc cacgcgcacg ggtactaccg 600

gtaccactct actaccagga tagtcaatga agcttaccta acttgaacct gtctttttac 660

cctcaattac catccagcta taaattttcc tctcccacga atccgatgct cgccactcgc 720

cgtgctcttc gctatttacg gctgtcgaaa cggctctcgc tttcaaatcc cccctcccaa 780

ttccgccata ttcacaccac acttcaaaac cgttcgaggg atgaggagga ggcagctcct 840

tctcgtccct tgttgttgcc cccccccgcg ataacgataa aaccgcctgt ggaggaatat 900

gttcgacaga tacgcgagca gtatggcgac tacctccccg ccggtctact ggaggagggc 960

gaatacagaa tctacgagag atactatggc cgtccaatcc gactgctgca gccgggcgag 1020

acgctccacc attttccacc tccaaagacg cccgcggagg aggccttata cgcacaggtt 1080

atccagcgaa tcgcccaccc ccaaggggta gggatggaag aaggagaaga aggggaaggg 1140

ggaggaggag ggggaggagg aggtgaggac cttcgtgcca atggttctcc accattcttt 1200

cggacacacc cgctcactta cgccggtcga ttcgagtcgt ctcagattcg acttccgcca 1260

tcggtaaggg acgggacaga gtctgtgatc gtggatgtcg ccgcctcgca tctagcggaa 1320

tcggcgcata agactctcgg cggtccacac ctccaattct cgcccgtata ctcgaggaag 1380

cggagtaagc acatgttgac tattcccttg aacccgatta acagtcgcat gtcaagtatg 1440

gatgccgacg tgtttctcac tgccgtgatg ccggggctgt atgcgcagtg cttgagttcg 1500

ttgaccgagc tgcggaaacg gttgggcggc gattgggtgc ttggcgggga tggggaaccg 1560

ggtgttagga atgtgcttga tgtggggggt gggggcgctg gggtacttgc ttggcggagt 1620

gttatcgaag tggaagagga ggtaaggaag gataggcttt ccggaagagc cgggaagccg 1680

ccacctggtt cggatggggg ggctctgcct gcggcggggg agaggagtgg gatgcgagct 1740

gttgttgtcg ccgggtccat cgctcttcgg gggaaggttt cgaggctctt ggacgatact 1800
ctgtttattc cccgaatgcc ggatatccat ttcgaaaacc taaaccacac accgggtggc 1860
ctggtggaga agaataaggc ccagccgaga aaactctacg atctgatcat tgccacaaac 1920
aatctcctcc cgatagtgaa gtcgttttac cgccgccaag ccattgaaca actctggtcg 1980
catctaaatc cgaacggcgg cgtccttttg atgatcgaaa gaggcacgcc aatgggcttc 2040
gaggccatcg cctacgcccg ctccgttatt ctagaggact acatcaagga cgttggcgga 2100
gcctttcaat ccctcccgac cacgaatgcc cccggttccg ccagcccccc tgtcgaaaag 2160
ggacccggcg ctataatcgc cccgtgcacg aaccacgagc aatgccccat gttcgtctct 2220
ggccctacgg atggcaccca acgcaaagat tattgccgct tctcacagcg ctatgagcgc 2280
cccggctatc tgcagaaact catggaggag tcatccaaaa accacgagga cctcgagtac 2340
tcatacgtcg ccttccgtcg tggtgtcgac caccgcaccg acacgaagaa caagatcagc 2400
ccaacaatag acgactttga cataatcccc gtcgacggcg aaccccacac gacagttcaa 2460
agcccatata cagtgtcaca actccgtaat tacagtctaa ccctcccgcg catcatcctc 2520
ccacccatca aacgcgacgg gcacgtcata ctcgacgtgt gcactccgac aggcttcatc 2580
gaacgctgga ttgtccctaa gagcgtcgga aaattggagt ttagagacgc gaggaagagc 2640
ggatggggtg atctctgggc ccttggagcg aaaagtcgca caaggcggaa cataaagatt 2700
ggcggtggcg acaagagatt taggtcaaag ttagttgcca tcaaggaccc tcatagcgat 2760
agagtgaggg ctattgtcaa ggacaagacg accgccttct tgaaggctcg gaagaaggag 2820
acaagggagg cgaggagggc cgcaaggctt cagaagaagc tagaggctag gatgaggcaa 2880
gacgaaatgg ttcgtcagca aaaggaggag aaggaaagga agcagcgtga gaagttaatg 2940
gcggagaagg gggccaggga ggtgcacgaa ggggttgttc agatgcatgg ggagaggaag 3000
ggaaagaaac ggctggagaa gcaaatgcgg gaaagggagt aggaggcgac gaaggggggg 3060
tgcaatcgca gttttgcgta atgtaaccgt actttcaggc cgccggtggg tggtgtgtat 3120
atatgaggct tcccgacttt tgtaaattat atttctagct ccgaaaaagc gttatacata 3180
acagccattg atggtggttc atgagttggt gcgtcattcg aaaggtcgag tgattgttgg 3240
tgaaaggata ccacgccccc ccaccattcc cgaatccgcc aatctcttcc atcgcccgtc 3300
cagcagttgt gagggtgggg ggacgtgtat cggtgtattt gtggtatact cgtgtgcagg 3360
caagtgctgt cggtggcggg gttggcagga gatggcatga tatcttgaga aattttctgc 3420
tagggggtgt ggtggcatat caacgtggca ttctctattg gaggattctg tcgtataccg 3480
ttggatgggc tttccataca ttcctgcctt aggccaatcc gcttttcacc ctccgcccgg 3540
ccgagccggc gcggtgtcct tcaaggcggg gcgtgggctc ggagaacggg tgtttgtcta 3600
tcgagtgatg ttttgtggtc ttactgatta tagttatcaa tacatccaca tataccagga 3660
gcttctgagc gattgcttgc ctgcaggcaa gtgatccgga cccaccacta ggtgggcccg 3720
gtacggggag cactgtctgt ctgttcgctt cacttgtgcg gttcgaatgc tttacctggt 3780

tgtgcgctct gagtacgaga aatgggcggg caagctagtc tgtctattca cgaagggcgc 3840
aatgaactat cacaaaccaa tcccggattc ttctactctt ttgtcccttt ctcacggagg 3900
ttctttccag tgagcttcgc ccggtctcgt ctcgtccgcc attgcgtggt tatttacagc 3960
ggatggctta atccgcaccc ttgtctatca tcgcaatacg gcattggctc tggcgaaact 4020
atatcgtgtc gttccgaggg agaaaaaaaa atcccttcat tctactttcc cttcgtccac 4080
tccgattcac tggcagatga aaccgtaccg cgcgctcggg ctctttggtc cctattttct 4140
caaccggcga gggcgtcgtt agtatggggg ttggaaggct cctttggctt gtttgtcgtg 4200
agtagggagg gcgctgtcat tgctgagtag cctttcagcg gggcctgcgg gggtgcttct 4260
ttccgatcgg tcttgcgagg ggtggtattg ctattgggcg gtgttggcca ttcggcggcg 4320
ttgatgaaat ttctatcggg ggttctgtgg cctctggata cgtggtgtcg tatatcttct 4380
ggcggtttct ctgctgctaa gtatgatatc gcactattcg tggggacctg tcattaagtc 4440
tttacatagt tgctcactat ggtcttagga atccttgcgc tatcgtacac cactttcctt 4500
ctatctgaac aacactctat atgtttatct atcgtacagg gggagaaagg taacgcgtgg 4560
gcaagaatgc acggctggag aaaataaaat atgataggat gaaaaccgca aggtataata 4620
aactagactt ttttgccaca gcgactagtt ccaaactcta cagtactcag ggggcaaacc 4680
cacgcccgac tataaaagca aatctccagg ggggctcgca tcagcacggt gacataagcg 4740
cattccgggc tcgtctcatg ggatctcctt cgactggaag gggtgatgcc taaggacgac 4800
tttcgtgggt agggtacccc ggaaaagaag gaacggtgat gccttcgatg aggtcctgtt 4860
ttataagcct taaatcgcgc taggggttaa tgctttgtgg gactgtagca gcgctaccac 4920
cttctgatgg ccgtgcctag atgcgaacga gagaggtgtt tgaccgtcat tgtttggcct 4980
gtcggggctg acatcttccc cgccgagtag taccttcacc acctccgcat gcccatccca 5040
agcagcgagc gagagcggtg tttggccgca atcatctggc ctgtcagggt tgacctcttc 5100
cctcatgagt aacattttca ccacttcttc atgtccatga aaggcagcat gagagagagg 5160
tgattggccg tggctgtctg gcttgtcggg attgacctct tcccgcatga gtagcattct 5220
caccacctct tcatatccgg accagacggc atgcgagagc ggtgttcggc aatagatatc 5280
tggcttgtca gggttgactt cttcccgccc gagaaagatt ttcactactc cctcatgtcc 5340
agaaccagcg gcatgcgaga gtggcgttcg gccgagattg tctggcttgt cggggttgac 5400
ctcttcccgc tcgagtaata atttcgcaac tccctcatac cccctccaag cagcattcga 5460
gagcggtgtt tggccgtcat tatctggctc gttgggatta acatcctccc gcccgagcag 5520
tattctcacc acttcctcat gtccaaacca agcagcgagc gagagcggtg tttggccgtc 5580
attgtccggc ttgtcggggt tgacctcttc ccgcccgagc agtattttca ccacttcctc 5640
atgtccacag gcagtagcat gtgagagcgg tgtctaggcc ttctccgttc gttatattac 5700
tataccatcc tactgatctc agcatggcct aacttagcca agaacccctc tgaaaccacc 5760
aggagtaacc tctagcaatg tcagccacaa tctcttaatg tccagaccct taccgagacc 5820

ggttgtcgca ttggatgact gcacctttgt tctggagttc catcttttaa acagctttcc 5880
cgagagctgc atacgccaca tcaaacgtcg tagtcctcct tgtccattca cctggatgca 5940
caagcggata gccgaacagg ggacattcat tggatcggct ggtagtgttt ccaaggcatt 6000
tggctttcct ggttggccgg gcagtgggtg aagctaccat acttctgaat tcacatttcg 6060
cctaacggcc tcctggccat tggatatccc cccaggacgg aaagaacggc ttctggatgg 6120
ccaggattaa ggtatggttt cccagtccgc cacacagatt tatggagatc atattaaatt 6180
cctcaatcag caaactacct tgattcacat atctgatcac aaagaattat tgttcagggc 6240
tcggatacca tacctctcta tcaaagaagc aatgtacaag agaggtgggg ttggaggacg 6300
gatgctgtaa cagaacccac atttcccagc tcaccctgtt tcattaattc ctttgttcgg 6360
aacttctccg acagtaaaaa aaaattcgcg gatcttttcg ccaggaatct tgggaaccta 6420
agggctctct cttcctgccc tgggggaaat cttattgtgt cataatatta ttatgtgtga 6480
taatattatg acattttcaa gataccggta atactattct cgggccctag tagggttgag 6540
ttgtacgtga cggcttcttg gcgtcactct attttttggg tcttggcatt tcccctcgtg 6600
acaggaggcc cactaggggg atccatcctt tcctccttct ttcctccgcc gacaaccgtc 6660
aagctccacc gacaaccgtc aagttccacc gacaaccgtc aagttcgaca gaaacattcg 6720
gcctttctac ttgcacctct ccactcacgt cgtttctttt tttctgccac tatgactgct 6780
ttgtccatgc tcaatatggc cgttgaagcc gggcctctta ttcaccaacg ttatgatggg 6840
atcggtctcg ccgagtatat tcccttgctc gccaatggaa cttttttctt tgatgggtcg 6900
gatggagccc cgctccaggt ccgtcatctc cttccagcag actggaagcc cgtcaatgga 6960
ccacttacgg tcttgttcga ttccgccccc cataccatta ttgggactca cttctctatc 7020
cacaatcatc ccatttacaa gcttaacgat ggttctatct ttgttgtctt ggacctggcc 7080
accaagcacg gttttgagtt taccaatcaa tctttcatgg acccccagct tgaggtctcc 7140
ggggaaaacc gtttcttgcg tctcgttacc aatgaccaag acttcgccta ccatggctct 7200
agtcttagca tgattcatga caagggcgtt aagcgccctg ccaatgcgta agtacaattc 7260
taccaatgta ccagtttcgt gtgctcatat agtcataggt ttatcttgtt cagaacggac 7320
aaaatgaagc ccctcaaggc tcagtacccg gagatgggca acaacgatat ctgtacgtgg 7380
ctctcatttt ccctttattt agatctctat tcttgctacc atttgcctaa cgttttaata 7440
gccaagattc tcggtcgcat gtggcagaat tcctcggatg aggtcaaaaa tgtttataag 7500
gtaaaacatc tcacctaact gttagttaga atcactaaca cattaatagg caagggccca 7560
tcagcttgcg gtttcccaca agttgatcaa cccggattac aaatactccc cgcgccgctc 7620
tcgcgaaatc atgaggaggg gctccgggaa taaaggtcct tcggcccgta tcgaccttca 7680
tcgtgctggt gttgcgaagc gtggaaaaaa gaggatcatt acccaccttg ggttcctcac 7740
tcaacgtttc gcgactcacc ccactgctaa caatctcggt gatatcatcg aggaagccga 7800
aaaggtcttg aaggcctttg gtatggtcaa ttgagtgatc tctacggagg gactcatcgg 7860

tacctgggtg gtatactttt aatgggaggg atgatcttgt ctatcaaagg tcattcatta 7920
tctcagtcga ctaaatttat gacctaaata gataaaaaat atcttttaaa accaaaatga 7980
ggggtccgaa cactacatca tacagtaaat atcaagttgt gaagcggaag agagccgact 8040
atgggaagag gcagtaagaa tttaattctg actaagtctg gaaagtttac acagcacttg 8100
cacagaagtt atgctatggt ggtgccatca ccggaaaaac acgccaaaaa ttcgggacct 8160
atggtgccgt gacttagttt gagcgcaagt acatggttcg acacaaaaga gtgttacgga 8220
gtaggggtgt tggggttgat tttggccatt agcgttatcg tatagattgt ttgaaaacac 8280
gactcgtatt gcttaacaag aatatggaga gagctatttt tttaattttt tttaattttg 8340
atagatgtat tggactggag gccaagcatc tgatcaattg gttatactat actcctattt 8400
gttcatagtt gactgaggcc ctgtttggct acaacggttc ctttctgttt ccccatacaa 8460
agcgaccgtt gcagaacggg agggcaatag cttttatatg agaaacggtt ccaaaatgaa 8520
acggaaccga ggaaattgtg atctagtttc ttggatgtgc cgctgctact gaaatttttg 8580
ttataaaggt atccgtttta tatctgactc ccttcacaca gaccccaatg gcgcgaatcc 8640
ccggagtact ttgctcatga aagtcgtcct gtcgtccttg ggcatcaccc cttccagtcg 8700
aaggagatcc catgagacga gcccggaatg cacttatatg tcaccatatg gacatgaacc 8760
tccgttgaga tttgctttta cagtcgggcg ttgttttatt tcccgagtac tttagagttt 8820
ggaaccagtt ctattgcttt gacaaagtct agtttattat accttgcagt tttcatctta 8880
tcacatttta ttttctctag ccgtacattc ttgccctcgc gtttttttct cttgcacaac 8940
tgatagacat ataagtgttc ttcagataga aggaaatggc gtacgatagc gcaaggattc 9000
ctaagaccat tgtgagaaac tatgtaaaga cttctgacag gtccccacga atagtgcaat 9060
atcatactta gcagcagaga aaccatcaag aaggtagact acagcacata gtcagaggcc 9120
acggaactcc tgagacataa gggtagggat aaccaccgta cacgagaaga acaattagaa 9180
taaaacagag ccattttata acaaatacta catgacaaag ggctaccaga atatattgat 9240
gcgcagaggc ccatgagaga gagcatagga tccctatttc ggtacccgta gtttaagagg 9300
gtgaaacatg ccctagatat tagtatattt tctagggaac tgctcttata tgatatgaaa 9360
aatatacttg tcctctggcg tatgtcacat ccaccctgca agcttaacct ctttgggtgc 9420
tcatcatagg caaggcccac tagtttcagg agccagtcag atattataca atagaatcta 9480
tagatcggag gtcctaccga tttggatgtg attcgggtaa gtctgggaca ttctgttaag 9540
attataccca ctaccgaagc cggaaagagt ccagatggct ggccaatatc cgaaggggca 9600
taggccaaag ctatcaggtg ctctggcaca gttgcacatg ctcaggcaat caaactttga 9660
agataccgta aaaacccttg ccgttcacta ccccaccggg ttgttaattt cctaatctcc 9720
gccgtattca gacataagcg cttaagcaga aatagtgctt tcctgacacg gtacagccta 9780
agccctgtgg aattgttggt tctcatgaac tagttttcgc aaccctgtta acatgtcgga 9840
cgcatgtcgt gcgctgatgc ggcgaactac agggatattg gagacctacg gagatatcga 9900

gtggaaagga tttttacagt attggtacat ggctgttaga ctctcttgcc ctccctcctt 9960
ttgctacgca cgatgaagaa tagaacaata ccggtaggtc agatcagacg acgactctgc 10020
ttggaataga gagatagaga aatagcatgg ctcgggcccc tgcgcccgtc agccacccgg 10080
accgggccag ggttgagatt tgaaagaaga tttcactggg tcagcccggc agcactggta 10140
cccagagccg cggatgtgcc agcctggggt tactcattcg gcgctcgaac ttcccccaat 10200
aacccctccc ttacggcgtc tcgcccaaga ctagtggcat agggccaaaa tactctgtat 10260
catatgatat catacgatat cgttgtccaa tgcaaaaaga ccgcaaaccc aaacctaatt 10320
gaaggttgtg ataccactac aaaagtccga ggctcaatat tcccacccaa tcccatgtca 10380
accccccaca aaaaattctc ctctcagcgc ccactccagt gtaccaaatt caacgatatc 10440
ttccacacaa tacacccccg ccttccttcc taaaatgccc ccaagtatcc ccccaactaa 10500
aaaaaaccga attcaatggc gatggctaac ttggaattgt aaaacgaaga tatcatgatc 10560
cgccgcgagg tgatgttccg ggctggaatg atgcttgtca atgaaatgga agccactgcc 10620
tggaaccctg gcaaccttat ggttgggatt gccatccata agaatccaag cgttcgtatg 10680
gttaaagcgg ttgagtcggc caaggacttg ctccgtgaag tcgctgtgag cgaccataaa 10740
ctgcgcaaaa tggatcgcat caatgatctt cagccactgg cgaatatcca taatacactt 10800
ttacagcttg gaggattaat ctctttgaaa caacatttct acgaggaact tcaaggtatc 10860
aaattacgcc ttcaggaact gcagagacac atgacagaca acattggcca ggccgttgca 10920
gaagttcatg acttcatctg tttcgtcact tttgtcaact tgatccaccc gcaaaactgc 10980
gtattaacct ccaaatccat caaggatgcg gagattctcg cctctaagaa ctcatccccc 11040
gaggcactcc aggcgtttgt tgaactccgt caggtttacg atctgcagac tcccgttcca 11100
gggtaccgtc taagcaaagc cagggagcta cctgcaatca ctaggtgcgc aatggagcaa 11160
tggcacaaag gttacttttc tcatgcagcc gcaactggaa tagtaagtaa aaaaacatct 11220
cttccatctc agcactcgta cctaataagc gccatcccac ttgatactaa tatcaaccca 11280
cagcaagtta tcccattcca gatcatattc ggatggtgga gtgagttcaa tttcctcatt 11340
tcaatttgat ggtgttatta gctgacatca agagatgttg tcttatagtt aattatcaac 11400
gcaagtttgt tagtagctct caagagaaaa tcactccgat tgtgcaagaa cttatcaact 11460
gcgcatatac ctcggaggcc agtgagccaa aagataagga cgagaaacta tggatgcaag 11520
aagataagga ggcttttaag gctgttacta acctagctat gtctatgact gaattgtgca 11580
aaaatatccc tctacccgag catgtcaaac cgaaccctca gaaagagaag cagttgccga 11640
tttacaccca gttcaaagtc gagcctcatg ttcctccgat cttccaagaa ttccagaagc 11700
tggtggctca aatttacgga ctttttgaag aaaactcgtg ctcaactttc actatcatga 11760
ccaaagcgta ttatccaaaa gtggagaact accttaggtt ctctgaaaaa gaccagacaa 11820
catcttctgt gaagcacttg aggtaagcta tcttttaagg ttccaggttt cttttgcact 11880
aaagctaatt ttcaacagcg ccgcgaccta ctttttcagt tcgctccgtg aggtcccagt 11940

tgtggttctc atcaataaga atgtaggggg tgtcgtgcca cgcaaggctg aggttccact 12000

ccttgatgct gttagcttgg ccggagtgga tcccactatt gccgatgaag agctcttggc 12060

ggagattact tgcaagaatt tgccacaagt tggcatgcac tgtaagtttt tcattatggt 12120

actactggtt tcggatatgt tctgatctct gaacgtaaca cagtccttgt caggtatcgc 12180

ggacaatctc accagtgccc gcgcaactaa attctactat ctctggtggc gtttgatcgc 12240

agcgctctat cagcgaaaag atgacaaatt tcgctatccg ggtagtcttt agatagttcc 12300

attcaaaatc aaactcttgt atcacaagat ttgccatatc tccatccaca ctttcattgt 12360

aaatttatat caaaaaaaaa aagaccccat ccagccccct ggaagaacag aggaaggagg 12420

ggactggttg agggcatttt tcttccctta tcaccaggta catggtgaac ctcccagtga 12480

ggttggggag tttttatttt acaaagggat ctaattcggg atcatggctt caggcccatt 12540

cggggtagcg attgataatt tagaggagta tgttattgtc cattggttcc actttgggga 12600

ggataggagt ttcttttatt atttgggttt tctaggccat ctttccgggt tcttaccgcg 12660

ctttttcccg cagtttgcga ataaattcaa agacccaaag aaaacaaagt gatttagacc 12720

ccatcaaaaa gggcacctag ctctgggaat gtaatta 12757

<210> 6
<211> 15256
<212> DNA
<213> Tuber melanosporum

<220>
<221> misc_feature
<222> (15085)..(15085)
<223> n is a, c, g, or t

<220>
<221> misc_feature
<222> (15109)..(15109)
<223> n is a, c, g, or t

<220>
<221> misc_feature
<222> (15121)..(15121)
<223> n is a, c, g, or t

<400> 6
gtgtgcgtgc ttgaagtgaa ttggagtaaa gtgaaggtgg tggtggtgaa agtggcgaat 60

atgagagtat caaaggagga ggtgagcaga gcattgagct tattagtagg ttcagagcga 120

gttgcgtgcg ctgtctctag ggttagagtt acagttagga tgcggcgttg tgtgtgtgtg 180

ggcgtgggtg cgtgggtgga tatgtgggtg tgtgtgttat tcttatcatc tgagtttgtt 240

gtcgtctttt caatttcact cgagtcgctg taggaggcag gagggagagt tgtacgagta 300

ctgtagatgt agagcacctg tatcatatta tcatactaca cacagtacag tactgcacga 360

gcacgagcac gaatacggtt tcagtgaatc tggagcttca ctcgtttgtg ttttttagct 420

tcgtgcactt ttgtcaatag agtaattatt taatatcatg atggttatta taatatatta 480

tcatacaggt ggtggagctg tatgataccc ttcgtatcat atccttatgc atcacacatc 540
ctttgcataa caaagtagct ggttttaggc ctacagtgcc cacgcgcacg ggtactaccg 600
gtaccactct actaccagga tagtcaatga agcttaccta acttgaacct gtctttttac 660
cctccctcaa ttaccatcca gctatcctct cccacgaatc cgatgctcgc cactcgccgt 720
gctcttcgct atttacggct gtcgaaacgg ctctcgcttt caaatccccc ctcccaattc 780
cgccatattc acaccacact tcaaaaccgt tcgagggatg aggaggaggc agctccttct 840
cgtcccttgt tgttgccccc ccccgcgata acgataaaac cgcctgtgga ggaatatgtt 900
cgacagatac gcgagcagta tggcgactac ctccccgccg gtctactgga ggagggcgaa 960
tacagaatct acgagagata ctatggccgt ccaatccgac tgctgcagcc gggcgagacg 1020
ctccaccatt ttccacctcc aaagacgccc gcggaggagg ccttatacgc acaggttatc 1080
cagcgaatcg cccaccccca aggggtaggg atggaagaag gagaagaagg ggaaggggga 1140
ggaggagggg gaggaggagg tgaggacctt cgtgccaatg gttctccacc attctttcgg 1200
acacacccgc tcacttacgc cggtcgattc gagtcgtctc agattcgact tccgccatcg 1260
gtaagggacg ggacagagtc tgtgatcgtg gatgtcgccg cctcgcatct agcggaatcg 1320
gcgcataaga ctctcggcgg tccacacctc caattctcgc ccgtatactc gaggaagcgg 1380
agtaagcaca tgttgactat tcccttgaac ccgattaaca gtcgcatgtc aagtatggat 1440
gccgacgtgt ttctcactgc cgtgatgccg gggctgtatg cgcagtgctt gagttcgttg 1500
accgagctgc ggaaacggtt gggcggcgat tgggtgcttg gcggggatgg ggaaccgggt 1560
gttaggaatg tgcttgatgt ggggggtggg ggcgctgggg tacttgcttg gcggagtgtt 1620
atcgaagtgg aagaggaggt aaggaaggat aggctttccg gaagagccgg gaagccgcca 1680
cctggttcgg atgggggggc tctgcctgcg gcgggggaga ggagtgggat gcgagctgtt 1740
gttgtcgccg ggtccatcgc tcttcggggg aaggtttcga ggctcttgga cgatactctg 1800
tttattcccc gaatgccgga tatccatttc gaaaacctaa accacatacc gggtggcctg 1860
gtggagaaga ataaggccca gccgagaaaa ctctacgatc tgatcattgc cacaaacaat 1920
ctcctcccga tagtgaagtc gttttaccgc cgccaagcca ttgaacaact ctggtcgcat 1980
ctaaatccga acggcggcgt ccttttgatg atcgaaagag gcacgccaat gggcttcgag 2040
gccatcgcct acgcccgctc cgttattcta aaggactaca tcaaggacgt tggcggagcc 2100
tttcaatccc tcccgaccac gaatgccccc ggttccgcca gcccccctgt cgaaaaggga 2160
cccggcgcta taatcgcccc gtgcacgaac cacgagcaat gccccatgtt cgtctctggc 2220
cctacggatg gcacccaacg caaagattat tgccgcttct cacagcgcta tgagcgcccc 2280
ggctatctgc agaaactcat ggaggagtca tccaaaaacc acgaggacct cgagtactca 2340
tacgtcgcct tccgtcgtgg tgtcgaccac cgcaccgaca cgaagaacaa gatcagccca 2400
acaatagacg actttgacat aatccccgtc gacggcgaac cccacacgac agttcaaagc 2460
ccatatacag tgtcacaact ccgtaattac agtctaaccc tcccgcgcat catcctccca 2520

cccatcaaac gcgacgggca cgtcatactc gacgtgtgca ctccgacagg cttcatcgaa 2580
cgctggattg tccctaagag cgtcggaaaa ttggagttta gagacgcgag gaagagcgga 2640
tggggtgatc tctgggccct tggagcgaaa agtcgcacaa ggcggaacat aaagattggc 2700
ggtggcgaca agagatttag gtcaaagtta gttgccatca aggaccctca tagcgataga 2760
gtgagggcta ttgtcaagga caagacgacc gccttcttga aggctcggaa gaaggagaca 2820
agggaggcga ggagggccgc aaggcttcag aagaagctag aggctaggat gaggcaagac 2880
gaaatggttc gtcagcaaaa ggaggagaag gaaaggaagc agcgtgagaa gttaatggcg 2940
gagaaggggg ccagggaggt gcwcgaaggg gttgttcaga tgcatgggga gaggaaggga 3000
aagaaacggc tggagaagca aatgcgggaa agggagtagg aggcgacgaa gggggggtgc 3060
aatcgcagtt ttgcgtaatg tarccgtact ttcaggccgc cggtgggtgg tgtgtatata 3120
tgaggcttcc cgacttttgt aaattatttc tagctccgaa aaagcgttat acataacagc 3180
cattgatggt ggttcatgag ttggtgcgtc attcgaaagg tcgagtgatt gttggtgaaa 3240
ggataccacc ccccccacca ttcccgaatc cgccaatctc ttccatcgcc cgtccagcag 3300
ttgtgagggt gggggacgtg tatcggtgta tttgtggtat actcgtgtgc aggcaagtgc 3360
tgtcggtggc ggggttggca ggagatggca tgatatcttg agaaattttc tgctaggggt 3420
gtggtggcat atcaacgtgg cattctctat tggaggattc tgtcgcatac ccttggacgg 3480
gctttccata cattcctgcc ttaggccaat ccgcttttcg ccctccgccc ggctgagccg 3540
gcgcagtgtc cttcaaggcg gggcgtgggc tcggagaaca cgtgtttgtc tatcgagtga 3600
tgttttgtga tcttactgat tatagttatc aatacatcca catataccag gagcttcggc 3660
ttgcatgata tcagcgaatg tagctcgagg acgtaaatct gatagttaca ctaaacgttg 3720
gataccacgc tggttcagag ctagctcctt ctccgctcga ggtagaggat taagttcacc 3780
gccctctgaa tggcatctga tgatgttccg ggcaacttct ctcggggcac ctgtgtcgct 3840
agcaattgta tgataacttt tgcctttctg catgaagctc aatcattcgw actctctcat 3900
cgcttgaggc cattcgccgc ttgcctgttt cttgaccttg aggtatggtg tatgatggga 3960
ggtgttagct cctataagga aatcgtggcc aagatactag tgtcgggtta ctaaaataac 4020
ctctgctgcc gagaccgttg cgaaagctgg gtagattctc tctacatatt ctagtagatc 4080
ttgacgctga tttacggatg gataaaaagg aattttatgt tttttttggt tttaaagata 4140
ccgagggcgt ttcattgatc ctagatacat aagtttaatt taagtctaca cgatctataa 4200
tacgctgaga atgcctccca actatgccct tgattcccgc cgctcccaca cggtcatacc 4260
gtaatacagt aacttcattt tttttttgta cccactagcc tctttaggcc ttcatgggag 4320
gtcatatcgg ctcacaatcc agtggaggat tctccaatag taggctgttt agataatgtt 4380
agaattgggc agtggcggaa tatgctgttc ggggaacttc tcacaaaaac gctgtatcca 4440
ccagaagtga aagtgttcat gtgcatctcg atctcgggct ggaggtgcgg gtcgagttga 4500
gagtctccat tctcctcagc gctagcgatg tcgaaagcat ggaggatgtt ggcggcttcc 4560

tcctcggtga gatcgagctg acggttgctg gaaacgtaaa cacgggtgat agtgaaactg 4620
gacaccccgg gtgcgggctt ggcacgaacc ttccttcttg cagcagcctt atcggccgct 4680
tccttgtcga tgggggcctt ggggacaaag ccagcttcac gcaagagctt ctcaggggta 4740
gtagggtgac cgtgtttcgc caattcggtc tcgataaatt cttcgagcac gaggccgggg 4800
tcaccacgat ccctagcagc agtataaaga gaggcgatgt tttgccacat atacttattg 4860
gtctcagcga cccagacatc acgagtgaga ttggaaattt cggcttgagt gagcccataa 4920
ccgttacagt acttagaaat ccaggctaat agatggttag aaatagaggc aaaataggga 4980
tgtgatttac ttacatctct gcgcaacgta gggattcaat gcacgaaggt gctgagaggg 5040
gggaatacgg gccaaggcct catcaccatt gacgataaca gccggaatag tggcatcctg 5100
ggccgtccac ggctgtccag gtttatagta tccgcaatac aaacgcagaa ccgacacaac 5160
ataagggact tcggggtcga gggggactcg cccttgatga cggacggttg attgaattgc 5220
accgagccac ttgtcgtagg gcaagagttc gccgctgaca gtgaaagtga caccaccggg 5280
gggttgaacg ataatggggg agttctcgtg ctggacccag atacacccga tcctgtcttg 5340
catgagaatg tacccacggt ccgaaaggta caaggcgcat cccaaggtaa cattacgcat 5400
aatgaggcgt ctctcgagcc tgttctccca ctccgaaacc ttaggggggg gggagaggtt 5460
aacgaccatg ctcagattga caggacgaga cgacattatg aaaggtgggg gggcggttgg 5520
agacgggaaa agaaatttgg accgacaaaa ggaggattgc gtatgggctt ttcgatgagt 5580
ggagattggc ctcagtggct gttggtggtg gaagaaagga gggtggaagg tctggcaact 5640
tttgaaggct acccgtcacg aagtgggatg ccaaggccca aaaaaaagag tgacgccaag 5700
aagctgtcac gtacaactta gccctactag ggtctatcat aatacatact atacgataat 5760
aaattattat gatattatca taagattttc cccagagcag gtagagagcc cttaggtttc 5820
caagattcct ggcggaaaga tcggggaaca tttttctttt tttacggtca gagttgctcg 5880
aatagaaaaa tgagtcaagg tatttgatga agatctgatc gacattcggg agatgtttag 5940
aacaaaggaa tttagtgggg atgagctggg aaatgtgggt tctgttacag tatctgtccc 6000
ccaatcttaa ctagccctct tgtacattgc ctctttggta aaggggtatg gtatctgagc 6060
cttgaacagt aattccttgc gataggatat atcgatcagg atagtttgct ggctggaaaa 6120
tttaatatag tcttcataaa atcgtgtgac ggactgggaa atcatacctt gagtttggcc 6180
atccggaagc ctctctttct gctatgaggg aatatcaaat agccaaaagg ccatggggtg 6240
aaatgagaat ttagaaatat gatagcggtc ccttgccacc aaaagacaaa tgcttcggca 6300
ataccatcac ccgatccggt ggatgtctcc atgttcggct atccgcttga gcatccaggg 6360
gaatagacaa ggaagactac cacatttgat gcggtgtgta cggctgccga gaaaactgtt 6420
tgaaagatgg aactccagaa caaaggatta actggtcaac gcaatacctg gttgccgcaa 6480
gagtctgaag attaaaggat tgtaactagt attgttggag gttacttcta gcaattctag 6540
aggggttttt ggtaaagata gattatactg atatcagtcg gatgatatgg cactacaacg 6600

aaagaaagtg gtctagacca tgagcgcaaa aactccaaaa agaatttcct gccgggaaat 6660
tagaatattt aaccggttta aaaccgtaag atttgctggt ccaggaccag cggcaaattg 6720
agagccattg tgagagcatc taagaactca accttcagaa ctccaggata agtaggatga 6780
taaaagggaa atggggttta gaatgggtgc ataaagcctt ttgattctgg attaagatat 6840
ggccagataa gggagtctga agcaatgtgc atttaatctc cgctcggact cctaggataa 6900
aaacggacat gacaagtaaa ttataatgag gaaaaaagct tgattggttg taggggtcga 6960
ttgtatgatc agaggtgggg gtactacaag tttatcgata taatattggg aataactgtg 7020
aaaatggttc cagaatatag ctgagtattt tacaaacatg cattagttgc atatgagata 7080
tcatagatac tagagtcgcc ttgttcagtc tcttggctgt ctttgaatat ctagatatac 7140
cgggaaagaa aatgggaaag aaaatatcat cggcttcttt agtaaactct tcaaagatag 7200
caaaaaccca acctctactt acagcctgag cctatagctt ggtatggtac tcatttaccc 7260
tttcttccac agagctatgc acttgcaata tttcttccaa ctgtttgttt aaaatccatg 7320
aggttcgaca tcaaatgaaa ggggtaattt ccacatgcac aaaacatata ttgtgtgaat 7380
acctacaatt gagcctgcat aaacacatga acccaaatca attcagtaaa ttaaaaccca 7440
tcaagtagtt ggtgatttag ctcatgaaat ttgtgaagtt tttttgagct aaagggaaaa 7500
gagaaatgtt acgcaaattt ttttgtcacc cttagctggt cggctgtcaa tgcttgatat 7560
tgtgatataa attactacag gttgtattgc gggaatgttg acatattgca gtacgacatc 7620
ttctaaggcc atatgcatat aagagattac tttatcgtac taagaaagcc aagacaatga 7680
tttctactac ttttttctat attctcttac ctcatattcc actatgattt acaagagtca 7740
tgccaaatca agtattgtgt gatacctggt gaaaaatatc catcaaagcc tcagagctgt 7800
aaatatcgca aattgtaagg tgagagggga atcacaatgc ataatattat ataaacacca 7860
caactgctct actgggggtg ccttatgcta ggactttcct acatgcgacc cattaacaga 7920
ttttaaaaaa gaaaaaaagt cactgaggat gccccactgc cacattaaag gagagagtag 7980
ttcttagatc ttgtctccct cagcttgctt agtgcacctg tggacagaaa ctcaggtgaa 8040
gatagagaaa gaggtatcat gaattttaat gcacagtatt cattgcaatt taattccggg 8100
cacactaagt tatttattca tttactcaat ccctactgca atccaagcta ctcttgtcct 8160
tgcccaggct atatcttgag ggcggggaac aaacttattc tcattgcacc ttctgggcgc 8220
tacgagcgaa agattgacta tttcatcaat tttaaatcct aaatacaagt atcatacaca 8280
tctactcaag tacaagaatc cttgtaacga ttggaacaaa acaaattctg ggactcttgt 8340
cggacactac gttaacgcta gtaagtaaca aaaaatcttt ttaatattag tagtgtgtgt 8400
gacatcctct tagattttgc gtgacacccc tgccgctact aggtcttagt actcgagtag 8460
tagaataata ggagtagcga ccttcaacga cagccaagcg atcaaaaaga gtatcgtact 8520
cgagtattca aatggaaaat ctaccttcct atgataccag gtgaggtcat tgcggtggtt 8580
caatacgacg atcaaatcct gcgattacgc ataacaagtg tgagtgctcg agaagagcgc 8640

gaatgcacag ctgcctggcc tgcgtgggtc tgacagatct gtccttgctc tatttatttg 8700
ttttgttttt cctgagcgca gcacttttta ccctatgagg tatttataat aatattatgg 8760
atattctgga ggaggttcca aaaaatatat cggaaatgca cttaggggca atggtactag 8820
gattccatcg cacactatcc gctaatagat atatctaggt ttctagtttc cataaatatc 8880
agacgagaaa ggcttaccaa aatgaccaag ggggttgggt ctgggagatg tctacggtgc 8940
gacgatagaa cggataaaag cgcagggcgg ggataaatcg agactcggga tggcagcttt 9000
gatgtggatc agtcatgcgc agcggctact cgggatggac gagctctccc acgccctggc 9060
agttgagctg aactccaccg acttcaacgc tagcaacatc tcttcgatgt cgacactggt 9120
gggttgcttc caagggctta ttaaggtgga caaggaagca tcagccgtgc aactggccca 9180
ctttactctc caagagtacc tttccgctca tcccgatatt ttctgcagac gccatgcggc 9240
aatggcagag atctgcttaa cctatctgaa ttcccggcaa atcaaggacc tttcgaccga 9300
ccatctcctg acaccccgga tgcacccttt ctagaatact gttccgtgca ctggggaatc 9360
catgcgaaaa cggaactctc ggatggcagg agatcgcttg cgctgaagct attctaggag 9420
tttgatggcc acatatccac agaatttctt ttagcgcaag tggagaattt aagttcccag 9480
gggcgtgata cctgacctcc gtccagtggg ttgtactgcg catcgttctt tggaatcgtc 9540
gaggttgtgg ctgctttgac atccgtggga tgttgggatg ccaaagaagg gggttgtttg 9600
gggtaaccgc cacttgcacg ggttcataac gggtatgggg aggtggtgga aatgctactc 9660
gggttggaac aggtccatcc cgacgagccg gataacgcga ccggacaccg ctctcacatg 9720
ttactgcctg tgggcatgaa gaagtggtga aaatactgct cctgtttgag cggggagagg 9780
ctagccacga aaagccggac aatgacggtc aaacaccact ctctttcgct gcttggtttg 9840
gacatgggga agtggtgaga atactacttg ggcggaagat gttaatccca atgagccaga 9900
taatgacggc caaacaccgc tctcgaatgc tgcttggagg ggtcatgaga gagttgcgaa 9960
aatattactc gggcgggaag aggtcaacct cgaagagcca gacgagctcg gccgggcgcc 10020
gctcttgcat gccgctggtc atggacatga gggagtagtg aaaattttct cgggcgggaa 10080
ggggtcaacc tcgaaaagct agacaattac tcccgaacac cgcttttgca tgccgtttgg 10140
tctagatatg aagaggtggt gagtgtgcta ctcatagggg gggtgtcgat ctcggcgaac 10200
cggacagcag cggccaaata ccgctctcgc atgctgccgc tcatgggcac gaggaggtgg 10260
tgaaaatgtt actcatgtgg gaagaggtca aacccgacag gccagatgat ttcggccaaa 10320
cactgcgctc gcttgctgct tgggatggac atgggggatg atgaaagtac tactcggccg 10380
ggaggatgtc aaaccggaca ggccagacga tagcggcaaa accctgctct agttggccgc 10440
cgggatgggc atgaggtagt ggtgaaaata ctactcgggc gcgaagttgt cagtcctgac 10500
aaaccagata atggcgacca aacacccctc tcgttcgcag ctgggaatga ccatcagaag 10560
gtggtggccc tgctacagtc ccacaaagcg ttaaccccta gcgcggttta aggtttataa 10620
aaaacaacct cttcgaaggc atcaccttcc ttttcttcca gagtactttg ctcgatgggg 10680

gtcatcctag ggcatcatcc tttccagtcg aaggagatcc catgagacga gcccggaatg 10740
cgcttatatg ccaccatgcg gatgtgaacc tccctgggga tttgctttta tagtcgggcg 10800
tggatttttt tttctgagta cggtagagtt tggaactagt tctatcgccg tgacaaagtt 10860
tagtttagtt tattacacct tgcagtcctc atcctatcat atttgatttc ctccagccgt 10920
acattcttgc cctcacgttt tccctctccc ttgcgcaatt gataaacata tagagtgttc 10980
atatagaagg aaagtggtgt acgagagggg aagggttctt aagaccaatg tgagcaacta 11040
tgtaaggact gaatgatagg tccccacgaa tagtgcgata tcatactcag cagcagagaa 11100
accatcaaga aggtaactac agcacatatc cagaggcccc agacctgtga cataaggata 11160
gggataaaca ccgtacacga gaaggacaat tagaataaaa cagagccatt ttataacaaa 11220
tactacatga caaagggcta ccagtatcta ttgatacgca gaggctcacg agagaaagta 11280
taagatcccg atttctgtac ccgtagttta agagggtgaa acatgcccta gatcttagta 11340
tatttttcta gggaaccgct cttataatat tataataata tgaaaaatat acttgtgctc 11400
tagcgtatgt cacatccacc ctgcaagctt aacctctttg ggtgctcatc ataggcgagg 11460
cccactagtt ccaggagcca gtcagatatt atacaatagt atctatagat cggaggccct 11520
accgatttgg ctgtgattcg ggtgagtctg agacattctg ttaagatatt acccactact 11580
gaagccggac agagtccaga tggcctcctg gccgatatcc gaaggggcat aggccaaagc 11640
tatcaggtgt tctagcacag ctgcacatgc tgaggaaatc aaactctgaa gataccgtaa 11700
aacaccttgc cgttcgctac cccaccaggt agctaatctc catattcgga cataagcgct 11760
taagcaggaa tagtgctttc ctgacacggt acagcctaag ccctgtggca ttgttggttc 11820
tcatgaacta gttttcgcaa ccctgttaac ttgtcggacg tatgatagca tgtcgtgcgc 11880
gacctacagg gatattggag acctatagta cagagatatc gaatcgaaag gatttttaca 11940
gtattggtac atggccgtta gactctcttg cccaccctcc ttttgctacc cacgatgaaa 12000
aatagggtgc cggtgggtca gatcaaacaa ctttgcttgg aatagagaga tagagaaaca 12060
gcatggctcg ggccactgct cccgtcagcc acccggaccg gtccagagtt gggatttgaa 12120
aggggatttc cctggattag tccggcagca ctgtacctaa agccacacgc tgtgccagcc 12180
tggggttact cactcggcac tcgaacctcc cccgataacc cctcccttac agctaaggat 12240
ttcraatata cgtatattat tacaatatac tgtataatat tattatacgt tgcttttata 12300
ccatacatac catacgtgta ctaaaataag taaccctaaa ttaatgaccc taaaaagtaa 12360
acgtcctgaa caaatatccc aactagaggt tggcatagca acttctatac ttaaatttaa 12420
tgtagatttt gtaattttca ctttacacct tcgacaaact taagatatta tttgggaggg 12480
tattcagaaa actagttact caaaattgac ttgaatttgt aactattgtt tttgcaataa 12540
aaagtattct tccccattta cgactcctac tagtgattgt tatccaatcg tggatttttg 12600
tggaaacaat tttcttctat caaaatttaa taatatcctt atctcgtaat attaaaccat 12660
taaaagacca gattttacgg tattctacaa aattgtataa atacgaaatt aatcctaact 12720

tacagcatct cgcctaaaac tagtagcata gggctaaaat actatgtatc atatgacacc 12780
acacgatatc gttgtcccat gcaaaaagac cgcaaaccca aacctaattg aaggttgtga 12840
tcccactaca aaggtcagag gatcaatatt cccacccgat cccatgtcca cccccacgaa 12900
aaaatcctct cagcacccac tccagtgtat ctaagttgac aatatcttcc acacatacac 12960
ccccaccttc cttgctaaaa tgcccccaag tatcccccca actaaaaatc tgaattcaat 13020
aacgatagct aacttggaat tgtagacgaa gatatcatga tccgccgcga gacgatgttc 13080
cgggccggaa taatgcttgt caatgaaatg gaagccactg cctggaaccc tggcaacctg 13140
atggttggga ttgccatcca taagaatcct agcgttcgta tggttaaagc ggtcgaatca 13200
gccaaggact tgctccgtga agttgctgtg agcgacaata aactgcgcaa gatggatcgc 13260
atcaatgatc ttcagccact ggcgaatatc cataatacac ttttacagct tggaggatta 13320
atctctttga aaaaacattt ctacgaggaa cttcgaggta tcaagttacg ccttcaggaa 13380
ctgcagagac acatgacaga caacattggc caggccgttg cagaggttca tgacttcatc 13440
tgtttcgtca cttttgtcaa cttgatccac ccgcaaaact gcatattaac ctccaaatcc 13500
atcaaggatg ccgagattct cgcctctaag aacccatccc ccgaggcaca tcaggtgttt 13560
gttgaactcc gtcaggttta cgatctgcag acccccgttc cagggtaccg tctaagcaag 13620
gccagggagt tacttgcaat cactaggtgc gcaatggagc aatggcacaa aggttacttt 13680
tctcatgcgg ccgcaactgg aatcgtaagt aaaaaaacat caacactcgt acctaataag 13740
cgccatccca catggtacta acatcaaccc acagcaagtt atcccattcc agatcatatt 13800
cggatggtgg agtgagttca atctccttat tccggtttga tggtgttatt agctgacatc 13860
aagagatgtt gtcttatagt taattatcaa cgcaagtttg ttagtagctc tcaagagaaa 13920
attactccga ttgtgcaaga acttatcaac cgcgcatata cctcggaggc cagtgagcca 13980
gaggatgagg acgagaaacc atggatgcaa gaagataagg aggcttttaa agctgttact 14040
aatctagcta tgtctatgac cgaattgtgc aaaaatatcc ctctacccga gcatgtcaaa 14100
ccaaaccctc agaaagagaa gcagttgccg atttacaccc agttcaaagt tgagcctcaa 14160
gttcccccgc tcttccatga attccagaag ctggtggctc aaatctacgg actttttgag 14220
gaaaactcgt gctcaacttt cactatcatg accaaagcgt attatccaaa agtggagaac 14280
taccttaggt tctctgaaca agaccagata acatcttctg tgaagcactt gaggtaagct 14340
atcttttaag gtggtaggtt tttttttaca ccaaagctaa ttttcaacag cgctgcgacc 14400
tactttttta cttcgctccg ggaggtccca gttgtggttc tcgtcaataa gaatgtacag 14460
ggtgttggcc catgcaaggt tgaggttccc ctccttgatg ctattagttt ggcctgctcg 14520
gatcccacca ttgccgatga agagctcttg gcggaaatta cttgcaagaa tttgccacaa 14580
gttggcatgc actgtaagtt tttcattatg atattactgg tttcggatat gttctaatct 14640
ctgaacctaa cacagtcctt atcaggtatc gcggacaagc tcacccgtgc ccgcgcaact 14700
aaattctact atctctggtg gcgtttgatc tcagcgctct atcagcgaaa aggtgacaaa 14760

ttttactatc tgggtagtct ttagatagtt ctatttaaaa tcaaactctt gtatcacaag 14820

atttgcaata tctccatcca cacattcgtt gtgaatttat atcaaaaaaa gaattgctct 14880

ccagacccca tccagccccc tggaagaaca gaggaaggag ggggctggtt gagagcattt 14940

tctttcctta tcaccaggta catggtgaac ctcccagtga ggttagggag tttttatttt 15000

acaaagggat ctaattcggg atcatggctt caggcccatt cggggtagcg attgataatt 15060

tagaggagta tgttattgtc cattngttcc actttgggga ggataggant ttcttttatt 15120

ntttgggttt tctaggccat ctttccgggt tcttaccgcg ctttttcccg cagtttgcga 15180

ataaatcaaa gacccaaaga aaacaaagtg atttagaccc catcaaaaag ggcacctagc 15240

tctgggaatg taatta 15256

<210> 7
<211> 25
<212> DNA
<213> Tuber melanosporum

<400> 7
caggtccgtc atctccttcc agcag 25

<210> 8
<211> 26
<212> DNA
<213> Tuber melanosporum

<400> 8
ccacatgcga ccgagaatct tggcta 26

<210> 9
<211> 25
<212> DNA
<213> Tuber melanosporum

<400> 9
caatctcact cgtgatgtct gggtc 25

<210> 10
<211> 26
<212> DNA
<213> Tuber melanosporum

<400> 10
tctcgggctg gaggtgcggg tcgagt 26

<210> 11
<211> 31
<212> DNA
<213> Tuber melanosporum

<400> 11
tggtatatgt ggatgtattg ataactataa t 31

<210> 12
<211> 26
<212> DNA
<213> Tuber melanosporum

<400>  12
agagatagag aaatagcatg gctcgg                                            26


<210>  13
<211>  29
<212>  DNA
<213>  Tuber melanosporum

<400>  13
gtgtgcgtgc ttgaagtgaa ttggagtaa                                         29


<210>  14
<211>  27
<212>  DNA
<213>  Tuber melanosporum

<400>  14
taattacatt cccagagcta ggtgccc                                           27